# FASCICULE DE BREVET EUROPEEN

(11) **EP 2 643 296 B1**
(45) Date de publication et mention de la délivrance du brevet: **17.09.2014**
(21) Numéro de dépôt: 11794429.8
(22) Date de dépôt: 24.11.2011
(51) Int. Cl.: C07D 209/14, C07D 209/08, G01N 33/574, G01N 33/533

(54) **DERIVES FLUORESCENTS DE CYANINES POLYAMINES EN TANT QUE SONDE DE DIAGNOSTIC**
FLUORESZIERENDE CYANINPOLYAMINDERIVATE ALS DIAGNOSTISCHE SONDE
FLUORESCENT CYANINE-POLYAMINE DERIVATIVES AS A DIAGNOSTIC PROBE

(30) Priorité: 24.11.2010 FR 1059687
(43) Date de publication de la demande: 02.10.2013
(73) Titulaire: Pierre Fabre Médicament, 92100 Boulogne-Billancourt (FR); Centre National de la Recherche Scientifique (CNRS), 75016 Paris (FR)
(72) Inventeur: GUMINSKI, Yves, F-81090 Lagarrigue (FR); IMBERT, Thierry, F-81290 Viviers les Montagnes (FR); PESNEL, Sabrina, F-45160 Olivet (FR); PILLON, Arnaud, F-31650 Saint Orens de Gameville (FR); LE PAPE, Alain, F-37130 Lignières de Touraine (FR); LERONDEL, Stéphanie, F-45000 Orléans (FR)
(74) Mandataire: Regimbeau
(86) Numéro de dépôt international: PCT/EP2011/070980
(87) Numéro de publication internationale: WO 2012/069607

(56) Documents cités:
- WO-A1-2009/013360
- WO-A1-2009/078970
- WO-A2-99/03823
- US-A- 5 968 479

## Description

La présente invention concerne des dérivés fluorescents de cyanines polyamines, leur procédé de préparation, les compositions diagnostiques les contenant et leur utilisation comme sonde de diagnostic du cancer.

Le cancer est encore une des principales causes de mortalité du monde occidental. Les moyens de lutte que sont la prévention, la chirurgie, la radiothérapie, l'immunothérapie et la chimiothérapie ne permettent pas encore, dans de nombreux cas, d'éradiquer la maladie.

Les raisons de cet échec reposent en partie sur la difficulté d'identifier et/ou de localiser la cellule tumorale et de la traiter sélectivement sans trop endommager le tissu sain.

L'imagerie par fluorescence *in vivo* est un outil permettant d'identifier les tissus tumoraux par rapport aux tissus sains, utilisant les nouvelles technologies d'imagerie optique. Cette technique utilisée avantageusement en chirurgie préclinique est en passe d'être développée au stade clinique. Cette approche de marquage par fluorescence est capable de détecter des tumeurs de taille extrêmement petite. Elle est basée sur l'obtention d'une cartographie des tissus *in vivo* utilisant l'émission de fluorescence d'un agent de contraste donnant ainsi une image tridimensionnelle par reconstruction avec un analyseur d'image.

Ces agents de contraste exogènes, appelés fluorophores, parcourent la circulation sanguine ou les canaux lymphatiques et viennent marquer des tissus cibles comme par exemple des tumeurs cancéreuses.

Les fluorophores organiques sont généralement des molécules aromatiques présentant un système d'électrons π fortement délocalisés leur conférant des propriétés de fluorescence dans la gamme du spectre visible.

Les tumeurs cancéreuses sont alors repérées soit simplement par accumulation des fluorophores due à une augmentation de la vascularisation autour de celle-ci, soit par l'expression de récepteurs ou transporteurs spécifiques sur lesquels viennent se fixer les fluorophores.

En effet, les cellules cancéreuses nécessitent pour leur prolifération un grand nombre d'éléments biologiques indispensables parmi lesquels sont présents les polyamines (spermine, spermidine, putrescine). Les polyamines sont indispensables à toute cellule en division. Les polyamines sont produites à partir des acides aminés que la cellule possède. L'arginine se transforme en ornithine, l'ornithine subit une décarboxylation pour fournir la putrescine. Cette putrescine (en C₄) peut s'allonger d'une chaîne en C₃ grâce à une adénosine méthionine, suivie d'une décarboxylation fournissant la spermidine. Cette dernière peut à nouveau s'allonger d'un motif en C₃ grâce aussi à un autre résidu méthionine fournissant la spermine. Ces polyamines peuvent également se dégrader par oxydation avec des polyamines oxydases et ainsi se retransformer en spermidine et putrescine. En situation normale, la synthèse endogène de polyamine suffit au catabolisme cellulaire mais dans le cas des cellules cancéreuses, la quantité disponible de ces polyamines, essentielles au développement et à la croissance de la cellule cancéreuse, n'est pas suffisante et un apport exogène de polyamines grâce à un transporteur de polyamines spécifique est le plus souvent nécessaire. Ce transporteur joue un rôle d'importation intracellulaire des polyamines naturelles que sont la putrescine, la spermidine et la spermine. Les cellules cancéreuses ayant besoin de quantités de polyamines plus importantes que les cellules saines, elles vont alors se pourvoir à l'extérieur, mettant en jeu l'activité du transporteur de polyamine.

Ainsi, la mise en évidence de ce transport actif permet de détecter les cellules cancéreuses et de suivre leur progression en termes de croissance et de dissémination. Cependant, le transporteur de polyamine, dans les cellules humaines, n'est, à ce jour, pas identifié au niveau moléculaire. La seule méthode pour démontrer sa présence est donc d'utiliser une sonde reconnue par ce transporteur qui, en s'accumulant dans la cellule, rendra compte de son activité. Ce transporteur joue en effet un rôle d'alimentation et de régulation.

Ce phénomène de transport des polyamines spécifique des cellules tumorales a été décrit grâce à un composé anticancéreux comprenant un motif « épipodophyllotoxine » lié à la spermine *(*Cancer Res. 2008, 68, 9845).

Dans le cas par exemple du composé de l'exemple 27 de la demande de brevet WO 2005/100363, le motif structural « épipodophyllotoxine » greffé sur la spermine ne semble pas modifier la reconnaissance du transporteur de polyamines mais permet d'internaliser le composé dans la cellule tumorale, qui ultérieurement jouera son rôle d'agent cytotoxique. Cependant, les tumeurs cancéreuses ont des phénotypes extrêmement variables et toutes n'expriment pas à un même niveau le transporteur de polyamines.

A l'aide des sondes de diagnostic, il est possible d'identifier par avance, c'est-à-dire avant traitement antitumoral, par imagerie par fluorescence, les foyers tumoraux qui seront sensibles au traitement et ceux qui ne le seront pas.

Plus la fluorescence sera forte, plus la tumeur en question sera sensible au traitement avec un anticancéreux ciblé (par exemple le composé de l'exemple 27 de la demande de brevet WO 2005/100363).

La mise en évidence de ce système de transport des polyamines permet donc de sélectionner les patients porteurs de tumeurs susceptibles d'être soignées par tout composé anticancéreux vectorisé par le système de transport des polyamines, quel que soit son mécanisme d'action.

Des agents de contraste portant un motif fluorescent conjugué avec un motif désoxyglucose ont déjà été utilisés en imagerie de fluorescence pour sélectionner les cellules cancéreuses exprimant le transporteur de glucose (GLUT) *in vitro* et *in vivo (*Bioconjug. Chem. 2006, 17, 662 ; Bioconjug. Chem. 2007, 18, 1303 et Analytical Biochemistry 2009, 384, 254-262)*.*

De même, la mise en évidence de cellules tumorales *in vitro* a pu être réalisée grâce à des sondes fluorescentes telles que celles décrites dans la demande de brevet WO 2009/013360 présentant un motif fluorescent conjugué avec une polyamine. Ces sondes présentent une fluorescence dans la zone de longueurs d'onde λ ~ 450-520 nm. Si cette zone du spectre est optimum pour les études cellulaires *in vitro,* cette partie du spectre n'est pas adaptée pour une détection par imagerie de fluorescence *in vivo* du fait de l'absorption des tissus et de l'eau. La meilleure zone d'utilisation se situe dans la partie rouge du spectre visible. Cette fenêtre du proche infrarouge (λ ~ 650-900 nm) correspond à une faible absorption des tissus, ce qui condamne l'utilisation des sondes présentant une fluorescence dans les longueurs d'ondes plus basses, telles que celles décrites dans WO 2009/013360. Cette fenêtre est également optimum pour une faible **absorption** de l'eau, qui devient très importante pour les longueurs d'onde λ > 1000 nm. De plus, l'accessibilité à des tissus situés dans des zones profondes représente un défi pour la sélection du fluorophore adapté *(*Nature Biotech. 2001, 19, 316 ; Current Opin. in Chem. Biol. 2002, 6, 642 ; Cancer Res. 2004, 64, 8009).

Toutefois, à la connaissance des inventeurs, il n'existe pas à ce jour de sonde fluorescente permettant la détection *in vivo,* par imagerie par fluorescence, de cellules tumorales exprimant le système de transport des polyamines.

Le problème que se propose de résoudre la présente invention est donc de proposer des composés fluorescents ciblant le système de transport des polyamines et qui permettent de réaliser de l'imagerie médicale par fluorescence de bonne qualité chez l'homme *in vivo.*

Après des essais infructueux (voir exemple comparatif 13) montrant que tous les fluorophores ne peuvent pas être utilisés, les inventeurs ont découvert de manière surprenante que des molécules hybrides conjuguées cyanines - polyamines permettent de répondre spécifiquement à cette problématique. Ces molécules sont en effet bien internalisées par le système de transport des polyamines et émettent une fluorescence suffisante pour être détectable facilement.

La présente invention se rapporte donc à des composés constitués d'un motif polyamine lié à un motif cyanine substitué.

Le motif cyanine substitué de ces molécules porte la fluorescence adaptée à l'imagerie *in vivo* tandis que la partie polyamine de ces molécules est reconnue par le système de transport des polyamines, permettant à la molécule d'être internalisée au sein de la cellule cancéreuse. Le tissu tumoral ainsi marqué pourra se différentier nettement du tissu sain par imagerie par fluorescence et un rapport entre tumeur et muscle (tissu sain) pourra être établi.

De manière surprenante, les composés de la présente invention participent peu aux réactions chimiques dans un environnement physiologique, par exemple *in vivo,* au cours de la mesure et ne conduisent pas à une perte des propriétés de fluorescence, c'est-à-dire une perte du signal, phénomène de blanchiment.

La mise en évidence du système de transport des polyamines permet alors de sélectionner les patients porteurs de tumeurs susceptibles d'être soignées par tout composé anticancéreux vectorisé par le système de transport des polyamines, quel que soit son mécanisme d'action.

Des dérivés de cyanine 5-5 conjuguée avec des polyamines ont été décrits dans Electrophoresis 2007, 28, 822, sans qu'aucune structure de ces dérivés ne soit reportée. De par la nature des substituants présents sur la partie cyanine, ces dérivés sont différents de ceux de la présente invention. Le dérivé de cyanine 5-5 non conjuguée est utilisé pour analyser et marquer le contenu en polyamine des érythrocytes et pour suivre le processus de cancérogénèse dans les érythrocytes. Les dérivés de cyanine 5.5 conjuguée avec des polyamines sont ainsi obtenus au cours de l'analyse, la conjugaison se faisant directement dans l'échantillon pour mettre en évidence la présence de polyamine dans les érythrocytes. Toutefois, l'utilisation qui est faite ici des dérivés de cyanine 5.5 conjuguée avec des polyamines, et qui est un des objets de la présente invention, est différente de celle de cet article et n'a nullement été évoquée auparavant.

La présente invention concerne ainsi des dérivés fluorescents constitués d'un motif polyamine sur lequel est lié un motif porteur de la fluorescence, une cyanine substituée, leur procédé de préparation, les compositions de diagnostique les contenant et leur utilisation dans la mise en évidence des tumeurs par imagerie de fluorescence, grâce au ciblage des transporteurs de polyamines. Elle concerne également le moyen de sélection de patients porteurs de telles tumeurs, pouvant être traités avec des produits anticancéreux vectorisés par le système de polyamines.

La présente invention a donc pour objet des composés fluorescents de formule (I) suivante : 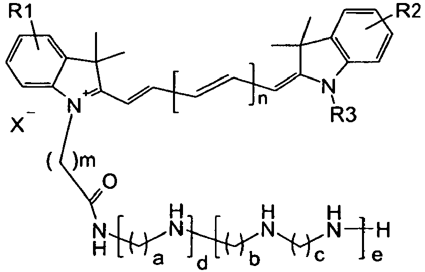 dans laquelle :
- R1 et R2 représentent chacun un atome d'hydrogène
- R3 représente un groupe C₁₋₆alkyle éventuellement substitué par un groupe -SO₃H,
- n vaut 1 ou 2,
- m vaut 3, 4, 5 ou 6,
- a, b, et c valent, indépendamment les uns des autres, 2, 3, 4, ou 5,
- d et e valent, indépendamment l'un de l'autre, 0 ou 1, à la condition qu'ils ne valent pas tous les deux simultanément 0, et
- X représente un atome d'halogène,
ou un de leurs sels pharmaceutiquement acceptables.

Par « atome d'halogène », on entend, au sens de la présente invention, les atomes de fluor, de chlore, de brome et d'iode. Il s'agira plus particulièrement d'un atome de brome, de chlore ou d'iode.

Par groupement «C₁₋₆alkyle», on entend, au sens de la présente invention, une chaîne hydrocarbonée saturée, linéaire ou ramifiée, comportant 1 à 6 atomes de carbone. A titre d'exemple, on peut citer les groupes méthyle, éthyle, propyle, isopropyle, butyle, isobutyle, sec-butyle, tert-butyle, pentyle ou encore hexyle.

Par groupement «C₁₋₄alkyle», on entend, au sens de la présente invention, une chaîne hydrocarbonée saturée, linéaire ou ramifiée, comportant 1 à 4 atomes de carbone. A titre d'exemple, on peut citer les groupes méthyle, éthyle, propyle, isopropyle, butyle, isobutyle, sec-butyle et *tert-*butyle*.*

Par groupement «C₁₋₄alcoxy», on entend, au sens de la présente invention, un groupe C₁₋₄alkyle, tel que défini ci-dessus, lié au reste de la molécule par l'intermédiaire d'un atome d'oxygène. A titre d'exemple, on peut citer les groupes méthoxy, éthoxy, propoxy, isopropoxy, butoxy, isobutoxy, sec-butoxy et tert-butoxy.

Par groupement « aryle », on entend, au sens de la présente invention, un groupement aromatique, comportant de préférence de 6 à 10 atomes de carbone, et comprenant un ou plusieurs cycles accolés, comme par exemple un groupement phényle ou naphtyle. Avantageusement, il s'agit du phényle.

Dans la présente invention, on entend désigner par « pharmaceutiquement acceptable » ce qui est utile dans la préparation d'une composition destinée à être administrée à un animal, tel qu'un mammifère, y compris l'homme, qui est généralement sûr, non toxique et ni biologiquement ni autrement non souhaitable et qui est acceptable pour une utilisation vétérinaire de même que pharmaceutique ou diagnostique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides organiques, tels que l'acide formique, ou des acides inorganiques tels que l'acide chlorhydrique ou l'acide bromhydrique, ou
(3) les sels formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique telle que l'hydroxyde de potassium ou l'hydroxyde de sodium.

Dans un autre mode de réalisation particulier de l'invention, R3 représente un groupe C₁₋₄alkyle, par exemple méthyle ou butyle.

Les composés de formule (I) selon l'invention pourront notamment être des composés pour lesquels m vaut 5.

En particulier, a, b et c pourront, indépendamment les uns des autres, prendre les valeurs 3 ou 4.

Dans un mode de réalisation particulier de l'invention, quand d vaut 0 et e vaut 1, a et b valent, indépendamment l'un de l'autre, 3 ou 4.

Dans un autre mode de réalisation particulier de l'invention, quand d vaut 1 et e vaut 0, a vaut 3 ou 4.

Dans encore un autre mode de réalisation particulier de l'invention, quand d vaut 1 et que e vaut 1 et a vaut 3, b vaut 4 et c vaut 3.

X représentera plus particulièrement un atome de brome ou d'iode.

Le sel pharmaceutiquement acceptable d'un composé de formule (I) pourra être un sel d'addition d'acide chlorhydrique, plusieurs molécules de HCl pouvant être incorporées dans le composé de formule (I) selon le nombre de fonctions amines (NH ou NH₂) qu'il comporte. Le sel pharmaceutiquement acceptable pourra donc être un mono-, di-, tri- ou tétra-chlorhydrate.

La présente invention a notamment pour objet les composés de formule (I) suivants :
1) iodure de 1-(6-(3-(4-(3-aminopropylamino)butylamino)propylamino)-6-oxohexyl) 2-((1*E*,3*E*,5*E*)-5-(1-méthyl-3,3-diméthylindolin-2-ylidène)penta-1,3-diényl)-3,3-diméthyl-3*H*-indolium,
2) iodure de 1-{5-[4-(3-aminopropylamino)-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
3) iodure de 1-{5-[3-(4-aminobutylamino)-propylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
4) iodure de 1-{5-[4-amino-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
5) bromure de 1-(6-(3-(4-(3-aminopropylamino)butylamino)propylamino)-6-oxohexyl)2-((1*E*,3*E*,5*E*)-5-(1-butyl-3,3-diméthylindolin-2-ylidène)penta-1,3-diényl)-3,3-diméthyl-3*H*-indolium,
6) bromure de 1-{5-[4-(3-aminopropylamino)-butylcarbamoyl]-pentyl}-3,3-diméthyl-2-{(1*E*,3*E*)-5-[1-butyl-3,3-diméthyl-1,3-dihydroindol-(2*E*)-ylidène]-penta-1,3-diényl}-3H-indolium,
7) bromure de 1-{5-[4-amino-butylcarbamoyl]-pentyl}-3,3-diméthyl-2-{(1*E*,3*E*)-5-[1-butyl-3,3-diméthyl-1,3-dihydroindol-(2E)-ylidène]-penta-1,3-diényl}-3H-indolium,
8) iodure de 1-(5-{3-[4-(3-aminopropylamino)butylamino]-propylcarbamoyl}-pentyl)-2-{(1E,3E,5E)-7-[1,3,3-triméthyl-1,3-dihydroindol-(2E)-ylidène]-hepta-1,3,5-triényl}-3,3-diméthyl-3H-indolium,
9) iodure de 1-{5-[4-(3-aminopropylamino)-butylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl} -3H-indolium,
10) iodure de 1-{5-[3-(4-aminobutylamino)-propylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl} -3H-indolium,
11) iodure del-[5-(4-aminobutylamino)-pentyl]-3,3-diméthyl-2-{(1E,3E,5E)-7-[1,3,3-triméthyl-1,3-dihydroindol-(2E)-ylidène]-hepta-1,3,5-triényl}-3H-indolium,
12) bromure de 1-(5-{3-[4-(3-aminopropylamino)butylamino]-propylcarbamoyl}-pentyl)-2-{(1E,3E,5E)-7-[1-butyl-3,3-diméthyl-1,3-dihydroindol-(2E)-ylidène]-hepta-1,3,5-triényl}-3,3-diméthyl-3H-indolium,
13) bromure de 1-{5-[4-(3-aminopropylamino)-butylcarbamoyl]-pentyl}-3,3-diméthyl-2-{(1E,3E,5E)-7-[1-butyl-3,3-diméthyl-1,3-dihydroindol-(2E)-ylidène]-hepta-1,3,5-triényl}-3H-indolium,
14) bromure de 1-{5-[3-(4-aminobutylamino)-propylcarbamoyl]-pentyl}-3,3-diméthyl-2-{(1E,3E,5E)-7-[1-butyl-3,3-diméthyl-1,3-dihydroindol-(2E)-ylidène]-hepta-1,3,5-triényl}-3H-indolium, et
15) bromure de 1-[5-(4-aminobutylcarbamoyl)-pentyl]-2-{(1E,3E,5E)-7-[1-butyl-3,3-diméthyl-1,3-dihydroindol-(2E)-ylidène]-hepta-1,3,5-triényl}-3,3-diméthyl-3H-indolium,
ou un sel pharmaceutiquement acceptable de ceux-ci, tel qu'un sel d'addition d'acide chlorhydrique.

L'invention a également pour objet une composition diagnostique comprenant au moins un composé de formule (I) selon l'invention ou un sel pharmaceutiquement acceptable de celui-ci et au moins un excipient pharmaceutiquement acceptable.

Par « composition diagnostique », on entend, au sens de la présente invention, une composition destinée à être administrée à un patient, notamment un être humain, en vue de permettre de réaliser un diagnostic, et plus particulièrement un diagnostic *in vivo.* Dans le cadre de la présente invention, il s'agira plus particulièrement de permettre la mise en évidence de tumeurs cancéreuses.

Les compositions diagnostiques selon l'invention peuvent préférentiellement être formulées pour une administration par voie parentérale, préférentiellement intraveineuse, destinée aux mammifères, y compris l'homme.

L'ingrédient actif peut être administré sous formes unitaires d'administration, en mélange avec des supports pharmaceutiques classiques, aux animaux ou aux êtres humains.

Pour une administration intraveineuse, on utilisera notamment des suspensions aqueuses, des solutions salines isotoniques ou des solutions stériles et injectables qui contiennent des agents de dispersion et/ou des agents mouillants pharmacologiquement compatibles.

Les composés de l'invention en tant qu'agent de diagnostic peuvent être utilisés à des doses comprises entre 0,01 mg et 1000 mg. La dose administrée peut être avantageusement comprise entre 5 mg et 500 mg, et notamment entre 10 mg et 200 mg. Il peut être nécessaire d'utiliser des doses sortant de ces gammes ce dont l'homme du métier peut se rendre compte lui-même.

L'utilisation d'un composé de formule (I) selon l'invention ou un sel pharmaceutiquement acceptable de celui-ci en tant que sonde de diagnostic pour imagerie médicale, notamment par imagerie par fluorescence, plus particulièrement pour mettre en évidence des tumeurs cancéreuses exprimant le système de transport des polyamines *in vivo* ou *in vitro,* et notamment *in vivo* est également décrite.

La présente invention concerne également les composés de formule (I) selon l'invention ou un sel pharmaceutiquement acceptable de ceux-ci pour leur utilisation dans le diagnostique, plus particulièrement in vivo, d'une tumeur exprimant le système de transport des polyamines, notamment par imagerie médicale, telle que par imagerie par fluorescence.

La présente invention concerne également l'utilisation d'un composé de formule (I) selon l'invention pour la préparation d'une composition diagnostique destinée à la mise en évidence d'une tumeur cancéreuse exprimant le système de transport des polyamines, plus particulièrement *in vivo,* notamment par imagerie médicale, telle que par fluorescence.

Une méthode de mise en évidence (méthode de diagnostique) *in vivo* d'une tumeur cancéreuse exprimant le système de transport des polyamines par administration d'une quantité suffisante d'un composé de formule (I) selon l'invention ou d'un sel pharmaceutiquement acceptable de celui-ci à une personne en ayant besoin est aussi décrite.

Cette administration est suivie d'une étape de détection de la fluorescence émise par le composé de formule (I) par imagerie médicale par fluorescence afin de visualiser la tumeur.

La présente invention a finalement pour objet un procédé de synthèse des composés de formule (I) selon l'invention comprenant les étapes successives suivantes :
(i) couplage entre un composé de formule (VII) suivante : 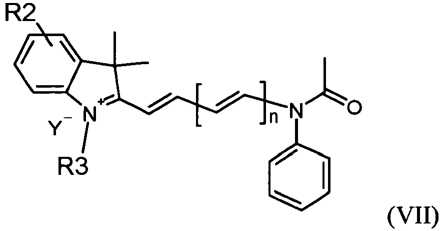 pour laquelle R2, R3 et n sont tels que définis précédemment, et Y représente un atome d'halogène, et en particulier un atome de brome ou d'iode, Y étant avantageusement identique à X,
   avec un composé de formule (V) suivante : 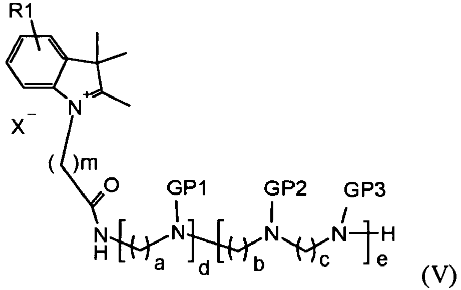 pour laquelle R1, X, m, a, b, c, d et e sont tels que définis précédemment et GP1, GP2 et GP3, identiques ou différents, représentent un groupe N-protecteur, pour donner un composé de formule (VIII) suivante : 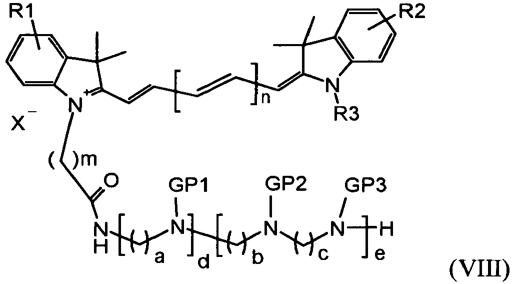 pour laquelle, R1, R2, R3, X, m, n, a, b, c, d, e, GP1, GP2 et GP3 sont tels que définis précédemment,
(ii) déprotection des fonctions aminés protégées par les groupements GP1, GP2 et GP3 dans le composé de formule (VIII) obtenu à l'étape (i) précédente pour donner un composé de formule (I) selon l'invention,
(iii) éventuellement salification du composé de formule (I) obtenu à l'étape (ii) précédente pour obtenir un sel pharmaceutiquement acceptable de celui-ci, et
(iv) séparation du composé de formule (I) ou de son sel pharmaceutiquement acceptable obtenu à l'étape précédente du milieu réactionnel.

Par « groupe N-protecteur », on entend, au sens de la présente invention, tout substituant qui protège le groupe NH ou NH₂ contre les réactions indésirables tels que les groupes N-protecteur décrits dans Greene, « Protective Groups In Organic synthesis », (John Wiley & Sons, New York (1981)) et Harrison et al. « Compendium of Synthetic Organic Methods », Vols. 1 à 8 (J. Wiley & sons, 1971 à 1996), cet ouvrage décrivant également les méthodes pour déprotéger ces groupements protecteurs. Les groupes N-protecteur comprennent les carbamates, les amides, les dérivés N-alkylés, les dérivés amino acétale, les dérivés N-benzylés, les dérivés imine, les dérivés énamine et les dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (BOC), le benzyloxycarbonyle (Cbz), le p-méthoxybenzyloxycarbonyle, le p-nitrobenzyl-oxycarbonyle, le trichloroéthoxy carbonyle (TROC), l'allyloxycarbonyle (Alloc), le 9-fluorénylméthyloxycarbonyle (Fmoc), le trifluoro-acétyle, les carbamates de benzyle (substitués ou non) et similaires. Il pourra s'agir en particulier d'un groupe Boc ou Alloc.

### Etape (i) :

Le couplage pourra être réalisé dans un solvant tel que l'éthanol, en présence d'acétate de sodium.

### Etape (ii) :

De préférence, les groupements GP1, GP2 et GP3 sont identiques et peuvent représenter un groupe BOC ou Alloc qui peuvent être déprotégés en milieu acide, notamment en présence d'acide chlorhydrique ou d'acide trifluoroacétique.

### Etape (iii) :

Cette étape pourra être réalisée par réaction du composé de formule (I) obtenu à l'étape (ii) avec un acide ou une base pharmaceutiquement acceptable. Il s'agira de préférence d'un acide pharmaceutiquement acceptable tel que l'acide chlorhydrique.

Selon la nature des groupements protecteurs GP1, GP2 et GP3 et le sel pharmaceutiquement acceptable désiré, il peut être envisagé d'effectuer les étapes (ii) et (iii) de manière « one-pot », c'est-à-dire dans un même réacteur, sans isoler l'intermédiaire de synthèse entre les deux étapes, et notamment en utilisant les mêmes réactifs (notamment le même acide pour déprotéger les groupements GP1, GP2 et GP3 et pour former le sel pharmaceutiquement acceptable).

### Etape (iv) :

Le composé obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Le composé de formule (V) peut être préparé par couplage peptidique entre un composé de formule (III) suivante : 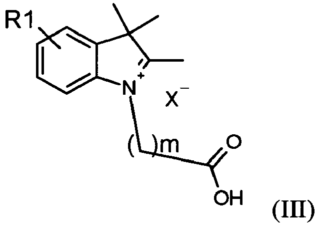 pour laquelle R1, m et X sont tels que définis précédemment, avec une polyamine protégée de formule (IX) suivante : 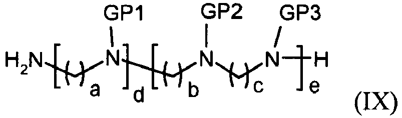 pour laquelle GP1, GP2, GP3, a, b, c, d et e sont tels que définis précédemment, ou un sel d'addition d'acide (organique ou inorganique comme décrit précédemment) de celle-ci comme un chlorhydrate.

Le couplage peptidique pourra être réalisé en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tetraméthyluronium hexafluorophosphate (HBTU), le 2-(1H-benzotriazole-1-yl)-1,1,3,3-tetraméthyluronium tetrafluoroborate (TBTU) ou encore le O-(7-azobenzotriazol-1-yl)-1,1,3,3-tétraméthyluronium hexafluorophosphate (HATU), éventuellement associé à un auxiliaire de couplage tel que le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HAt) ou le N-hydroxysylfosuccinimide (sulfo NHS). En particulier, le couplage pourra être réalisé en présence de TBTU.

Le couplage pourra en outre être réalisé en présence d'une base telle que la triéthylamine. Un solvant inerte tel que l'acétonitrile pourra être utilisé.

Les polyamines sont disponibles dans le commerce et peuvent être protégées par des techniques bien connues de l'homme du métier permettant d'avoir accès facilement aux composés de formule (IX). Un exemple de dérivé polyamine, la spermine protégée par trois groupements BOC, est décrit dans Tetrahedron Lett. 1998, 39, 439.

Les composés de formule (III) peuvent être préparés à partir d'une indoline de formule (IIa) suivante : 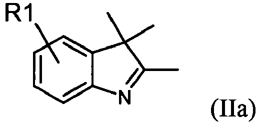 pour laquelle R1 est tel que défini précédemment,
par réaction avec un acide de formule (X) suivante : 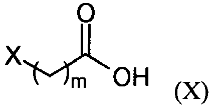 pour laquelle m et X sont tels que définis précédemment.

Ce couplage est réalisé notamment sans solvant, par simple mélange des deux réactifs de formules (IIa) et (X), plus particulièrement à température élevée, notamment entre 80 et 150°C.

Les indolines de formule (IIa) et les acides de formule (X) sont soit disponibles dans le commerce, soit facilement accessibles par des techniques bien connues de l'homme du métier.

Le composé de formule (VII), quant à lui, peut être obtenu par couplage entre un composé de formule (IV) suivant : 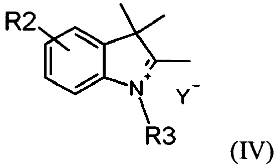 pour laquelle R2, R3 et Y sont tels que définis précédemment,
et un composé de formule (VI) suivante : 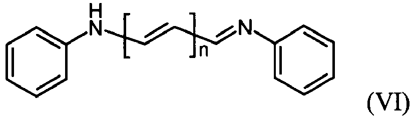 pour laquelle n est tel que défini ci-dessus, ou un sel d'addition d'acide (organique ou inorganique comme décrit précédemment) de celui-ci tel qu'un chlorhydrate.

Ce couplage peut être réalisé en présence d'anhydride acétique et d'acide acétique, notamment au reflux.

Les composés de formule (IV) peuvent être préparés à partir d'une indoline de formule (IIb) suivante : 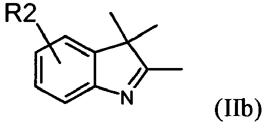 pour laquelle R2 est tel que défini précédemment,
par réaction avec un réactif de formule R3-Y pour lequel R3 et Y sont tels que définis précédemment.

Ce couplage est réalisé notamment sans solvant, par simple mélange des deux réactifs, plus particulièrement à température élevée, notamment entre 80 et 150°C.

Les indolines de formule (IIa) et les réactifs de formule R3-Y sont soit disponibles dans le commerce, soit facilement accessibles par des techniques bien connues de l'homme du métier.

Les composés de formule (VI) peuvent être préparés par réaction de l'aniline, produit disponible dans le commerce, avec un diacétal de formule (XI) suivante : 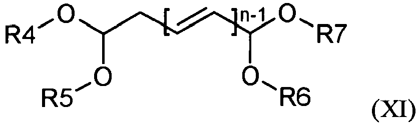 pour laquelle n est tel que défini précédemment et R4, R5, R6 et R7, identiques ou différents, représentent un groupe C₁₋₆alkyle, de préférence C₁₋₄alkyle tel que méthyle, ou (R4 et R5) et/ou (R6 et R7) forment ensemble une chaîne -(CH₂)ₚ- avec p représentant 2 ou 3.

En particulier, R4, R5, R6 et R7 sont tous identiques et représentent notamment un groupe C₁₋₄alkyle tel que méthyle.

L'aniline sera utilisée à raison d'au moins deux équivalents molaires par rapport au diacétal, c'est-à-dire qu'au moins deux moles d'aniline sont utilisées pour chaque mole de diacétal utilisée.

Le diacétal de formule (XI) est soit disponible dans le commerce, soit accessible par des techniques bien connues de l'homme du métier, notamment à partir du dialdéhyde correspondant.

La synthèse des composés de formule (I) est notamment présentée sur le schéma général suivant :

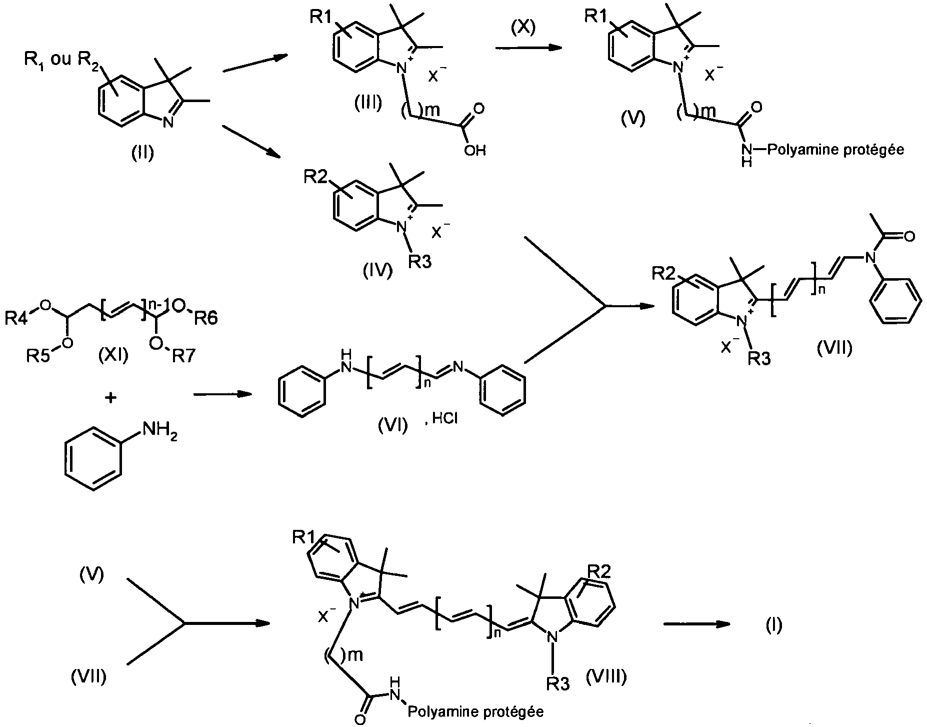

Les exemples suivants décrivent les différents stades pour la préparation des composés de l'invention et servent uniquement à illustrer l'invention et ne sont en aucune manière, limitatifs.

### FIGURE

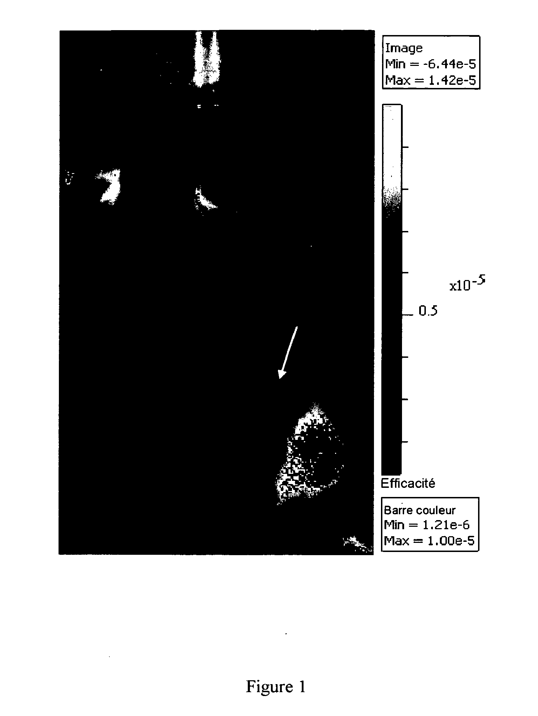

La Figure 1 représente l'image de flurorescence d'une souris possédant une tumeur, 24 heures après l'injection de la sonde F98942 (exemple 7) (50 µg) par voie intraveineuse. La flèche indique la tumeur.

### EXEMPLES

Les abréviations suivantes ont été utilisées dans la partie expérimentale qui suit :
- APCI: Ionisation chimique à pression atmosphérique
- BOC: *tert*-Butyloxycarbonyle
- CCM: Chromatographie sur Couche Mince
- DMSO: Diméthylsulfoxyde
- ESI: Ionisation par électrospray
- HPLC: Chromatographie Liquide Haute Performance
- Rdt: Rendement
- Rf: Rapport frontal
- RMN: Résonance Magnétique Nucléaire
- TBTU: 2-(1H-Benzotriazole-1-yl)-1,1,3,3-tetraméthyluronium tetrafluoroborate
- THF: Tétrahydrofurane

### I - Synthèse des composés selon l'invention

### Exemple 1 :

### Bromure de 1-(6-(3-(4-(3-aminopropylamino)butylamino)propylamino)-6-oxohexyl)2-((1E,3E,5E)-5-(1-butyl-3,3-diméthylindolin-2-ylidène)penta-1,3-diényl)-3,3-diméthyl-3H-indolium.

Composé de formule (I) avec R1 = R2 = H, R3 = nBu, n = 1, m = 5, X = Br, d = e = 1, a = 3, b = 4, et c = 3.

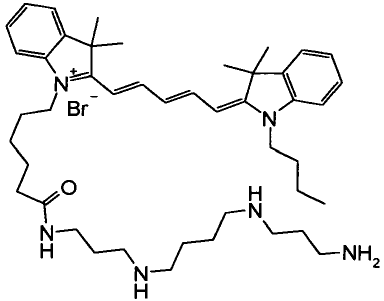

***Stade* 1**- Préparation du chlorhydrate de la triBOC-spermine *(*Bioorg. Med. Chem. 2002, 10, 2397). Composé de formule (IX) avec P = COOtBu, a = 3, b = 4, c = 3, et d = e = 1.

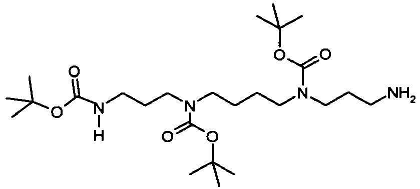

40 g de spermine sont placés en solution dans 300 mL de CH₂Cl₂ et refroidis à 0°C. On ajoute goutte à goutte 23,8 mL de trifluoroacétate d'éthyle en solution dans 50 mL de CH₂Cl₂. La solution sous agitation est laissée ensuite 1 heure à température ambiante. Le résidu obtenu est repris dans 600 mL de THF, on y introduit 140,28 mL de triéthylamine. Une solution de 174,48 g de BOC₂O dans 200 mL de THF est alors introduite en maintenant la température à 20°C. L'agitation à température ambiante est maintenue trois heures supplémentaires, puis le milieu réactionnel est jeté sur de l'eau et extrait par l'éther isopropylique. Après décantation, séchage sur sulfate de sodium, filtration et évaporation, la solution organique est évaporée. Le résidu obtenu est alors repris dans 900 mL d'un mélange MeOH / H₂O 8/2. On ajoute 192,6 g de carbonate de Césium et on porte le tout à reflux pendant trois heures. Après évaporation du méthanol sous pression réduite, on ajoute de l'eau et on extrait par de l'éther isopropylique. On décante la phase organique, puis on introduit dans cette phase organique de l'acide chlorhydrique 0,5 N. On laisse décanter. Il apparaît 3 phases. La phase du milieu sous forme huileuse contient le produit attendu sous forme de chlorhydrate. On décante cette phase, puis on la reprend par du CH₂Cl₂, et on sèche sur sulfate de sodium. Après filtration et évaporation, on obtient 54 g du chlorhydrate de la triBOC spermine sous forme d'une huile incolore utilisée directement dans l'étape suivante (Rdt = 50 %).
CCM SiO₂ : CH₂Cl₂-MeOH-NH₄OH (90-10-10) - Rf = 0,5.
C₂₅H₅₀N₄O₆, HCl : 539,17 - (Masse APCI+500°C MH⁺ = 503,4).

***Stade 2**-* Préparation du bromure de 1-(5-carboxypentyl)-2,3,3-triméthyl-3H-indolium *(*Bioorg. Med. Chem. 2006, 4, 92-97). Composé de formule (III) avec R1 = H, m = 5, et X=Br.

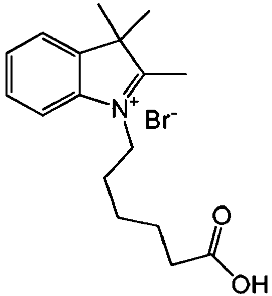

1,6 g de 2,3,3-triméthylindolénine et 2 g d'acide bromohexanoïque sont portés à fusion 3 heures à 120°C. On observe la prise en masse du milieu. Le milieu réactionnel est cristallisé dans l'acétate d'éthyle, filtré, lavé à l'acétone puis à l'éther isopropylique. Après séchage sous vide, on obtient 2,3 g de solide rosâtre (Rdt = 64.6 %).
C₁₇H₂₄NO₂: 274,32 - (Masse ESI+400°C M = 274,1).

***Stade 3-*** Préparation du bromure de 1-(9,14-bis(tert-butoxycarbonyl)-2,2-diméthyl-4,19-dioxo-3-oxa-5,9,14,18-tétraazatétracosan-24-yl)-2,3,3-triméthyl-3H-indolium. Composé de formule (V) avec R1 = H, m = 5, et X = Br, la polyamine protégée correspond au composé de formule (IX) avec P = COOtBu, d = e = 1, a = 3, b = 4, et c = 3.

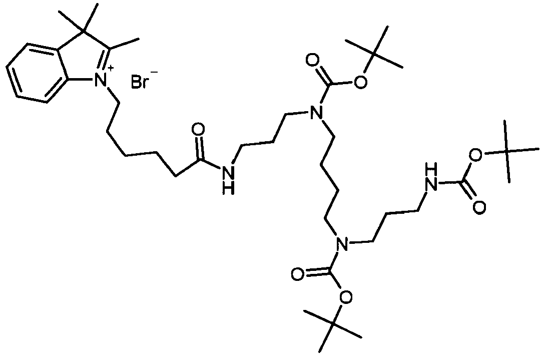

Au mélange de 1 g de chlorhydrate de tri-BOC-spermine obtenue au stade 1 précédent, 0,5 g du composé de la préparation du stade 2 précédent et de 0,6 mL de triéthylamine en solution dans 30 mL d'acétonitrile est ajouté en une fois à température ambiante 0,6 g de TBTU. On laisse en contact à température ambiante environ 3 heures. Le milieu réactionnel est hydrolysé avec une solution saturée en NaHCO₃ puis on extrait à l'acétate d'éthyle. La phase organique après décantation est lavée avec une solution HCl 1N, décantée et séchée sur sulfate de sodium anhydre. Après filtration et évaporation sous pression réduite, le résidu obtenu est fixé sur silice pour être purifié par flash chromatographie (gradient heptane 100 % à acétate d'éthyle 100 %). Les fractions concernées par le produit sont évaporées sous pression réduite pour donner 1.2 g d'huile brune (Rdt quantitatif).
C₄₂H₇₂BrN₅O₇ : 838,98 - (Masse ESI+400°C M = 758,6).

***Stade 4-*** Préparation du bromure de 1-butyl-2,3,3-triméthyl-3H-indolium. Composé de formule (IV) avec R2 = H, R3 = *n*Bu, et X = Br.

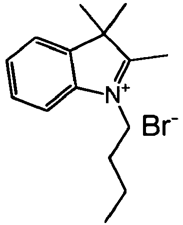

8 g de 2,3,3-triméthylindolénine et 6,9 g de bromobutane sont portés à fusion 8 heures à 110°C. Le milieu réactionnel gommeux obtenu est cristallisé dans l'acétate d'éthyle sous agitation pendant 1 heure, filtré, lavé à l'éther isopropylique. Après séchage sous vide, on obtient 7 g de solide rouge violacé (Rdt = 47 %).
C₁₅H₂₂BrN : 296,26 (Masse trouvée : APCI+500°C M= 216,2).
RMN ¹H (400MHz, DMSO d6) δ = 8.01 (1H, dd,H-7), 7.85 (1H, dd, H-4), 7.63 (4H,H-5, H-6), 4.47 (2H, t, H-1'), 2.86 (3H, s, CH3), 1.82 (2H, m, H-2'), 1.55 (6H, s, 2 x CH3) 1.44 (2H, m, H-3'), 0.94 (3H, t, H-4').

***Stade 5-*** Préparation du chlorhydrate de3-(phénylamino)allylidène)aniline *(*Bioorg. Med. Chem. 2006, 4, 92-97). Composé de formule (VI) avec n = 1. 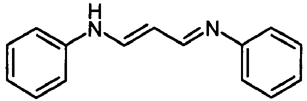 24,7 g d'aniline en solution dans 300mL d'HCl 0,75 N sont ajoutés goutte à goutte à 21,8 g de 1,1,3,3-tétraméthoxypropane en solution dans 300mL d'HCl 0,75 N. On observe l'apparition d'une coloration orange intense et la précipitation d'un solide cristallin orange. Le milieu est ensuite laissé sous agitation 3 heures à 50°C. Le milieu refroidi est filtré, séché sous vide pour donner 15,2 g de solide orange (Rdt = 44,2 %).
C₁₅H₁₄N₂, HCl - (Masse ESI+400°C MH⁺ = 223,1).

***Stade 6-*** Préparation du bromure de 1-butyl-3,3-diméthyl-2-((1E,3E)-4-(N-phénylacétamido)buta-1,3-diényl)-3H-indolium. Composé de formule (VII) avec R2 = H, R3 = *n*Bu, X = Br, et n = 1.

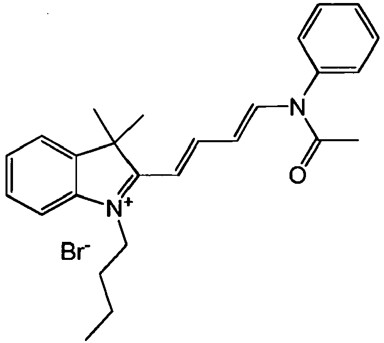

Un mélange de 3 g de la préparation du stade 4 précédent avec 5,2 g de la préparation du stade 5 précédent en solution dans 45 mL de mélange 2/1 d'anhydride acétique et d'acide acétique est porté au reflux 2 heures. On évapore sous pression réduite le solvant puis on fixe le résidu obtenu sur de la silice afin de purifier par flash chromatographie (gradient sur silice : acétate d'éthyle 100 % à acétate d'éthyle / méthanol 90/10). On évapore les fractions concernées pour obtenir 3,6 g d'huile rouge scarabée qui cristallise (Rdt = 77,7 %).
C₂₆H₃₁BrN₂O : 467,454.

***Stade* 7-** Préparation de bromure de 1-(9,14-bis(tert-butoxycarbonyl)-2,2-diméthyl-4,19-dioxo-3-oxa-5,9,14,18-tétraazatétracosan-24-yl)-2-((1E,3E, 5E)-5-(1-butyl-3, 3-diméthylindolin-2-ylidène)penta-1,3-diényl)-3,3-diméthyl-3H-indolium. Composé de formule (VIII) avec R1 = R2 = H, R3 = *n*Bu, X = Br, n = 1, et m = 5, la polyamine protégée correspond au composé de formule (IX) avec P = COO*t*Bu, d = e = 1, a = 3, b = 4, et c = 3.

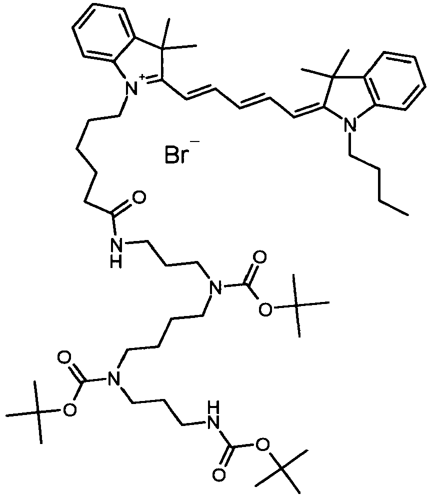

Un mélange de 2,4 g de la préparation du stade 3 précédent et de 1,5 g de la préparation du stade 6 précédent en solution dans 40 mL d'éthanol en présence de 1,4 g d'acétate de sodium est laissé sous agitation à température ambiante environ 1 heure. Après ajout de silice, le produit est fixé sur celle-ci par évaporation sous pression réduite de l'éthanol. On purifie le produit par flash chromatographie SiO₂ (gradient : acétate d'éthyle 100 % à acétate d'éthyle 75 % - méthanol 25 %). Les fractions concernées sont évaporées sous pression réduite pour obtenir 1,5 g de solide bleu scarabée (Rdt = 48 %).
C₆₀H₉₃BrN₆O₇ : 1090,35 - (Masse APCI+500°C M = 1009,9).

***Stade 8**-* Préparation du trichlorhydrate de bromure, de 1-(6-(3-(4-(3-aminopropylamino)butylamino)propylamino)-6-oxohexyl)2-((1*E*,3*E*,5*E*)-5-(1-butyl-3,3-diméthylindolin-2-ylidène)penta-1,3-diényl)-3,3-diméthyl-3*H*-indolium. Composé de formule (I) avec R1 = R2 = H, R3 = *n*Bu, n = 1, m = 5, d = e = 1, a = 3 , b = 4, c = 3, et X = Br.

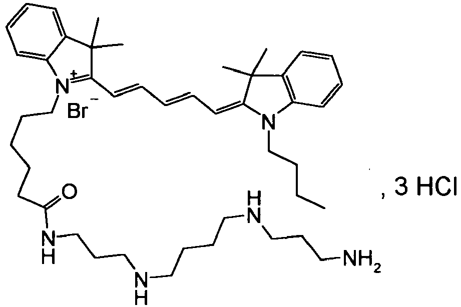

1,5 g de la préparation 7 précédente en solution dans 100 mL de dichlorométhane est laissé en contact 2 heures sous agitation à température ambiante en présence de 10 mL d'une solution HCl / dioxane 4M. Le solvant est évaporé sous pression réduite pour obtenir un résidu qui est cristallisé dans l'acétate d'éthyle. Après filtration et séchage sous vide, on obtient 1 g de solide bleu intense sous forme de trichlorhydrate.

On réalise une purification par HPLC préparative sur colonne X-bridge C-18 10 µ, 30x250 mm, λ : 220 nm, débit : 40 mL /min. Phase mobile gradient : HCl 5 mM 100 % à HCl 5 mM 80 % - acétonitrile 20 %. Les fractions concernées sont lyophilisées après avoir éliminé l'acétonitrile sous pression réduite. Le lyophilisat est repris dans l'acétate d'éthyle, filtré et séché sous vide pour conduire à 440 mg de solide violet intense (Rdt = 35,6 %).

Pureté HPLC analytique : 98,5 % colonne C8-X-bridge 5 microns 4.6x250 mm. λ: 220 nm - phase mobile 60% tampon KH₂PO₄ 6.8g/L pH 4 - CH₃CN 40 %. Temps rétention : 7.56 min.
RMN ¹H (400MHz, D₂O) δ = 8.015 (2H, t, J = 12.8 Hz, H-14, H-14'), 7.53 (2H, m, J = 7.2 Hz, H-6, H-6'), 7.42 (H-5, 5'), 7.26 - 7.33 (4H, m, H-3,H-3',H-4, H-4'), 6.51 (1H, t, J = 12.4 Hz, H-15'), 6.26(1H, d, J = 13.6 Hz, H-13), 6.23 (1H, d, J = 13.6 Hz, H-13'), 4.08 (4H, m, H-10, H-10'), 2.85 - 3.18 (12H, m, H-7",H-9", H-11", H-14", H-16", H-18"), 2.25 (2H, t, J = 7.2 Hz, H-4"), 2.10 (2H, quin, J = 7.4 Hz, H-17"), 1.78 - 1.85 (8H, m, H-1", H-8", H-11, H-13"), 1.64 (12H, s, CH3 x4), 1.37-1.48 (4H, m, H-2", H-3"), 1.15 (2H, d, H-12), 0.97 (3H, t, H-13)
C₄₅H₆₉BrN₆O, 3 HCl : 899,39 (Masse APCI +500°C : M = 709,5).

### Exemple 2 :

### Iodure de 1-(6-(3-(4-(3-aminopropylamino)butylamino)propylamino)-6-oxohexyl) 2-((1E,3E,5E)-5-(1-méthyl-3,3-diméthylindolin-2-ylidène)penta-1,3-diényl)-3,3-diméthyl-3H-indolium.

Composé de formule (I) avec R1 = R2 = H, R3 = Me, X = I, n = 1, m = 5, d = e = 1, a = 3, b = 4, et c = 3.

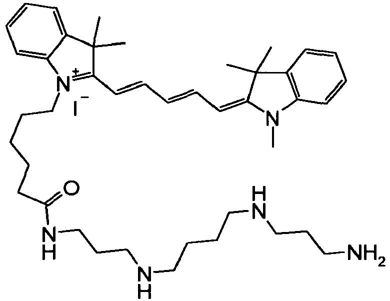

***Stade 1*** *:* Préparation de l'iodure de 1-méthyl-3,3-diméthyl-2-((1E,3E)-4-(N-phénylacétamido)buta-1,3-diényl)-3H-indolium *(*Bioorg. Med. Chem. 2006, 4, 92-97). Composé de formule (VII) avec R2 = H, R3 = Me, X = I, et n = 1.

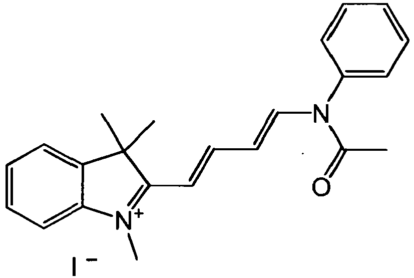

Un mélange de 2 g d'iodure de tétraméthyl indolénium avec 3,4 g de la préparation du stade 5 de l'exemple 1, en solution dans 30 mL de mélange 2/1 d'anhydride acétique et d'acide acétique est porté au reflux 2 heures. On évapore sous pression réduite le solvant puis on fixe le résidu obtenu sur de la silice afin de purifier par flash chromatographie (gradient sur silice : acétate d'éthyle 100% à acétate d'éthyle / méthanol 90/10). On évapore les fractions concernées pour obtenir 2 g de solide noir (Rdt = 64 %).
C₂₃H₂₅N₂O : 472,361 - (Masse ESI +400°C : M = 345,2).

***Stade 2 :*** Préparation de l'iodure de 1-(9,14-bis(tert-butoxycarbonyl)-2,2-diméthyl-4,19-dioxo-3-oxa-5,9,14,18-tétraazatétracosan-24-yl)-2-((1E,3E,SE)-5-(1-butyl-3,3-diméthylindolin-2-ylidène)penta-1,3-diényl)-3,3-diméthyl-3H-indolium. Composé de formule (VIII) avec R1 = R2 = H, R3 = Me, X = I, n = 1, et m = 5, la polyamine protégée correspond au composé de formule (IX) avec P = COOtBu, d = e = 1, a = 3, b = 4, et c = 3.

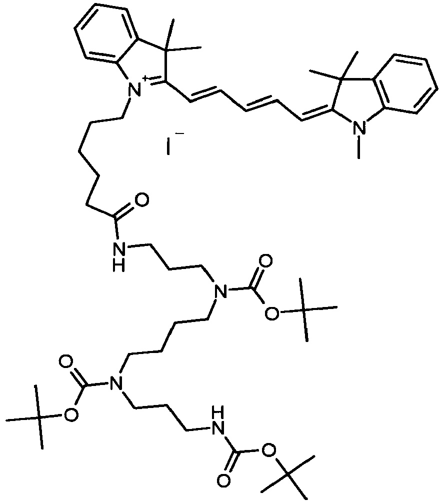

Un mélange de 0,17 g de la préparation du stade 3 de l'exemple 1 et de 0,1 g de la préparation du stade 1 de cet exemple, en solution dans 15 mL d'éthanol en présence de 0,2 g d'acétate de sodium est laissé sous agitation à température ambiante environ 4 heures. Après ajout de silice, le produit est fixé sur celle-ci par évaporation sous pression réduite de l'éthanol. On purifie le produit par flash chromatographie SiO₂ (gradient : acétate d'éthyle 100 % à acétate d'éthyle 75 % - méthanol 25 %). Les fractions concernées sont évaporées sous pression réduite pour obtenir 0,1 g de solide bleu scarabée (Rdt = 45 %).
C₅₇H₈₇IN₆O₇ : 1095,24 - (Masse ESI+400°C M = 967,6).
CCM SiO₂ : CH₂Cl₂ / CH₃OH / Heptane 5 / 1 / 1 - Rf : 0,65.

***Stade 3*** Préparation du trichlorhydrate du l'iodure de 1-(6-(3-(4-(3-aminopropylamino)butylamino)propylamino)-6-oxohexyl)2-((1*E*,3*E*,5*E*)-5-(1-méthyl-3,3-diméthylindolin-2-ylidène)penta-1,3-diényl)-3,3-diméthyl-3*H*-indolium. Composé de formule (I) avec R1 = R2 = H, R3 = Me, X = I, n = 1, m = 5, d = e = 1, a = 3, b = 4, et c = 3.

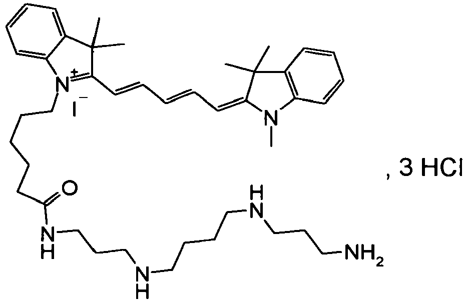

0,5 g de la préparation du stade 2 de cet exemple en solution dans 65 mL de mélange dichlorométhane / éthanol 3 / 1, est laissé en contact 1 heure sous agitation à température ambiante en présence de 2 mL d'une solution HCl / dioxane 4M. Le solvant est évaporé sous pression réduite pour obtenir un résidu bleu intense sous forme de trichlorhydrate.

On réalise une purification par HPLC préparative sur colonne Sunfire C-18 5 µ, 19 x 250 mm, λ : 220 nm, débit : 20 mL /min. Phase mobile gradient : HCl 5 mM 100 ù à HCl 5 mM 85 % - acétonitrile 15 %. Les fractions concernées sont lyophilisées après avoir éliminé l'acétonitrile sous pression réduite. Le lyophilisat est repris dans un mélange dichlorométhane / éther isopropylique 1 / 1, filtré et séché sous vide pour conduire à 130 mg de solide bleu intense. R(dt = 29,4 %).

C₄₂H₆₃IN₆O, 3 HCl : 857,314 - (Masse ESI+180°C M = 667,6).

Pureté HPLC analytique : 96,7 % colonne C8-X-bridge 5 microns 4.6x250 mm. λ : 220 nm - phase mobile 60 % tampon KH₂PO₄ 6.8g/L pH 4 - CH₃CN 40 %. Temps rétention : 7.37 min.

CCM SiO₂ : CH₂Cl₂ / Méthanol / NH₄OH 90 / 10 /1 + cuve ammoniaque - Rf : 0,15.

RMN ¹H (500MHz, DMSO-d6) δ = 9.25 (2H, br. s., H-15"), 9.09 (2H, br. s., H-10"), 8.34 (2H, t, J = 13.0 Hz, H-14', 14), 8.15 (4H, br. s., H-19"), 8.09 (1H, t, J = 5.6 Hz, H-6"), 7.61 (2H, d, J = 7.3 Hz, H-6', 6), 7.39 (4H, d, J = 2.4 Hz, H-4', 4, 3', 3), 7.20 - 7.29 (2H, m, H-5', 5), 6.58 (1H, t, J = 12.4 Hz, H-15'), 6.32(1H, d, J = 13.7 Hz, H-13), 6.28 (1H, d, J = 14.0 Hz, H-13'), 4.09 (2H, t, J = 7.2 Hz, H-10'), 3.60 (3H, s, H-10), 3.09 (2H, q, J = 6.6 Hz, H-7"), 2.93 - 3.02 (2H, m, H-16"), 2.88 (8H, s, H-14", 11", 9", 18"), 2.08 (2H, t, J = 7.3 Hz, H-4"), 1.99 (2H, quin, J = 7.4 Hz, H-17"), 1.74 - 1.82 (2H, m, H-8"), 1.68 (12H, s,H-11, 12, 11', 12'), 1.71 (6H, s, H-1", 13", 12"), 1.48 - 1.60 (2H, m, H-3"), 1.29 - 1.42 (2H, m, H-2")

RMN¹³C (126MHz, DMSO-d6) δ = 173.3 (C-9), 172.5 (C-9'), 172.3 (C-5"), 154.1 (C-14', 14), 142.8 (C-2), 142.1 (C-2'), 141.1 (C-7), 141.1 (C-7'), 128.5(C-4), 128.4 (C-4'), 125.4 (C-15'), 124.8 (C-5), 124.7 (C-5'), 122.5 (C-6'), 122.4 (C-6), 111.1 (C-3, 3'), 103.4 (C-13), 103.1 (C-13'), 48.9 (C-8', 8), 46.0 (C-14", 11"), 44.6 (C-9"), 43.8 (C-16"), 43.3 (C-10'), 39.9, 39.8, 39.6, 36.2 (C-18"), 35.7 (C-7"), 35.2 (C-4"), 31.2 (C-10), 27.2 (C-12', 12), 27.0 (C-11', 11), 26.8 (C-1"), 25.9 (C-8"), 25.8 (C-2"), 25.0 (C-3"), 23.6 (C-17"), 22.6 (C-12"), 22.6 (C-13").

**De** façon identique à l'exemple 2, mais à partir des matières premières correspondantes *(*J. Org. Chem. 2004, 69, 3530 ; Synthesis 2002, 2195 ; Synthesis 1994, 37 ; J. Org. Chem. 1978, 44, 1166), les produits des exemples 3 à 5 sont préparés.

### Exemple 3 :

### Iodure de 1-{5-[3-(4-aminobutylamino)-propylcarbamoyl]-pentyl}-3,3-diméthyl-2-{(1E,3E)-5-[1,3,3-triméthyl-1,3-dihydroindol-(2E)-ylidène]-penta-1,3-diényl}-3H-indolium. Composé de formule (I) avec R1 = R2 = H, R3 = Me, X = I, n = 1, m = 5, d = 0,e=1,b=3,etc=4.

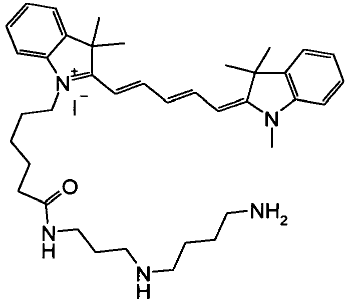

C₃₉H₅₆N₅OI = 737,81.

### Exemple 4 :

**Iodure de 1-{5-[4-(3-aminopropylamino)-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium.** Composé de formule (I) avec R1 = R2 = H, R3 = Me, X = I, n = 1', m = 5, d = 0,e=1,b=4,etc=3.

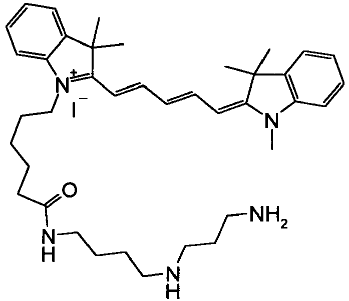

C₃₉H₅₆N₅OI = 737,81.

### Exemple 5 :

**Iodure de 1-{5-[4-amino-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium.** Composé de formule (I) avec R1 = R2 = H, R3 = Me, X = I, n = 1, m = 5, d = 1, a = 4, et e = 0)

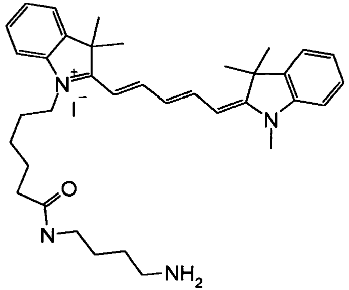

C₃₆H₄₉N₄OI = 680,72.

De façon identique à l'exemple 1 mais à partir des matières premières correspondantes ***(***J. Org. Chem. 2004, 69, 3530 ; Synthesis 2002, 2195 ; Synthesis 1994, 37 ; J. Org. Chem. 1978, 44, 1166), le produit de l'exemple 6 est préparé.

### Exemple 6 :

**Bromure de 1-{5-[4-(3-aminopropylamino)-butylcarbamoyl]-pentyl}-3,3-diméthyl-2-{(1*E*,3*E*)-5-[1-butyl-3,3-diméthyl-1,3-dihydroindol-(2E)-ylidène]-penta-1,3-diényl}-3H-indolium.** Composé de formule (I) avec R1 = R2 = H, R3 = Me, X = I, n = 1, m = 5, d = 0, e = 1, b = 4, et c = 3.

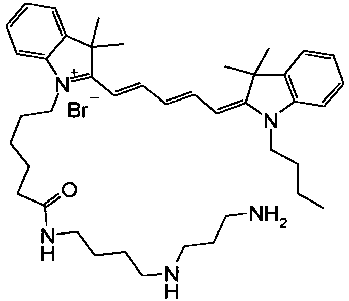

C₄₂H₆₂BrN₅O = 732,90.

### Exemple 7 :

**Bromure de 1-(5-{3-[4-(3-aminopropylamino)butylamino]-propylcarbamoyl}-pentyl)-2-{(1E,3E,5E)-7-[1-butyl-3,3-diméthyl-1,3-dihydroindol-(2E)-ylidène]-hepta-1,3,5-triényl}-3,3-diméthyl-3H-indolium.** Composé de formule (I) avec R1 = R2 = H, R3 = *n*Bu, X = Br, n = 3, m = 5, d = e = 1, a = 3, b = 4, et c = 3.

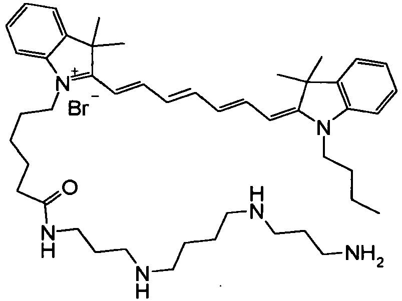

***Stade 1***- Préparation du bromure de 1-butyl-3,3-diméthyl-2-((1E,3E,5E)-6-(N-phénylacétamido)hexa-1,3,5-triényl)-3H-indolium. Composé de formule (VII) avec R2 = H, R3 = *n*Bu, n = 3, et X = Br.

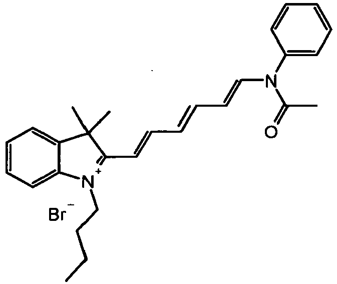

Un mélange de 3 g de la préparation du stade 4 de l'exemple 1, avec 5,7 g de chlohydrate de glutacondianyle en solution dans 45 mL de mélange 2/1 d'anhydride acétique et d'acide acétique est porté au reflux 2 heures. On évapore sous pression réduite le solvant puis on fixe le résidu obtenu sur de la silice afin de purifier par flash chromatographie (gradient sur silice : acétate d'éthyle 100% à acétate d'éthyle / méthanol 90/10). On évapore les fractions concernées pour obtenir 2,6 g d'huile rouge scarabée (Rdt = 52 %).
C₂₈H₃₃BrN₂O : 493,49 ( masse APCI+500°C MH⁺ = 413,2).

***Stade 2**-* Préparation du bromure de 1-(9,14-bis(tert-butoxycarbonyl)-2,2-diméthyl-4,19-dioxo-3-oxa-5,9,14,18-tétraazatétracosan-24-yl)-2-((1E,3E,5E,7E)-7-(1-butyl-3,3-diméthylindolin-2-ylidène)hepta-1,3,5-triényl)-3,3-diméthyl-3H-indolium. Composé de formule (VIII) avec R1 = R2 = H, R3 = nBu, X = Br, n = 3, et m = 5, la polyamine protégée correspond au composé de formule (IX) avec P = COOtBu, d = e = 1, a = 3, b =4,etc=3.

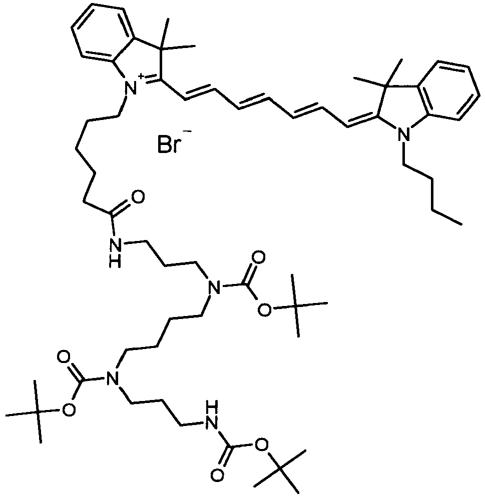

Un mélange de 1,2 g de la préparation du stade 3 de l'exemple 1 et de 4,2 g de la préparation du stade 1 précédent de cet exemple, en solution dans 50 mL d'éthanol en présence de 1,2 g d'acétate de sodium est laissé sous agitation à température ambiante environ 2 heures. Après ajout de silice, le produit est fixé sur celle-ci par évaporation sous pression réduite de l'éthanol. On purifie le produit par flash chromatographie SiO₂ (gradient : acétate d'éthyle 100% à acétate d'éthyle 75% - méthanol 25%). Les fractions concernées sont évaporées sous pression réduite pour obtenir 1,28 g de solide vert scarabée (Rdt = 46,2%).
C₆₂H₉₅BrN₆O₇ : 1116,39 (masse APCI+500°C M = 1035,6).

***Stade 3-*** Préparation du trichlorhydrate de bromure de 1-(5-{3-[4-(3-aminopropylamino)butylamino]-propylcarbamoyl}-pentyl)-2- {(1E,3E,5E)-7-[1-butyl - 3,3-diméthyl-1,3-dihydroindol-(2E)-ylidène]-hepta-1,3,5-triényl}-3,3-diméthyl-3H-indolium. Composé de formule (I) R1 = R2 = H, R3 = *n*Bu, X = Br, n = 3, m = 5, d = e =1,a=3,b=4,etc=3.

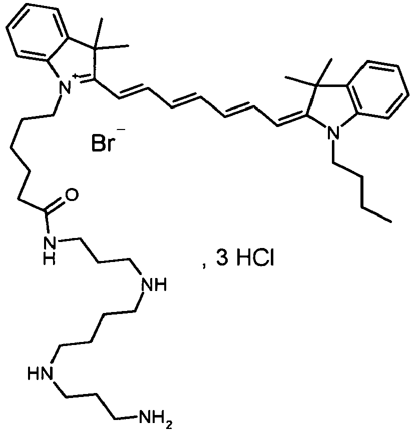

1,2 g de la préparation du stade 2 précédent de cet exemple, en solution dans 100 mL de dichlorométhane est laissé en contact 2 heures sous agitation à température ambiante en présence de 10 mL d'une solution HCl / dioxane 4M. Le solvant est évaporé sous pression réduite pour obtenir un résidu vert intense qui est cristallisé dans l'acétate d'éthyle. Après filtration et séchage, on obtient 0,95 g de solide vert intense du produit sous sa forme trichlorhydrate (Rdt = 29,4 %).
C₄₇H₇₁BrN₆O, 3 HCl : 925,43 - (Masse ESI+400°C : MH/2 = 368,4 et M = 607,5). Pureté HPLC analytique : 97,4 % colonne C8-X-bridge 5 microns 4.6x250 mm. λ: 750 nm - phase mobile 60 % tampon KH₂PO₄ 6.8g / L pH 4 - CH₃CN 40 %. Temps rétention : 12.15 min.

De façon identique à l'exemple 7 mais à partir des matières premières correspondantes, les produits des exemples 8 à 11 sont préparés.

### Exemple 8 :

**Iodure de 1-(5-{3-[4-(3-aminopropylamino)butylamino]-propylcarbamoyl}-pentyl)-2-{(1E,3E,5E)-7-[1,3,3-triméthyl-1,3-dihydroindol-(2E)-ylidène]-hepta-1,3,5-triényl}-3,3-diméthyl-3H-indolium.** Composé de formule (I) avec R1 = R2 = H, R3 = Me, X = I, n = 3, m = 5, d = e = 1, a = 3, b = 4, et c = 3.

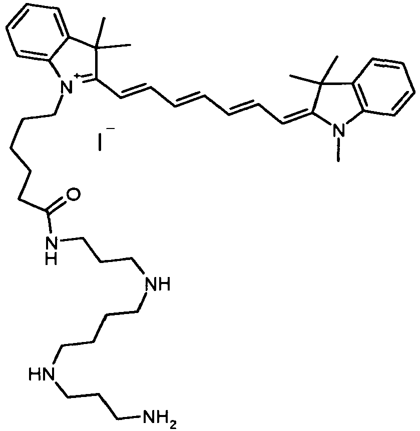

C₄₄H₆₅IN₆O = 816,95.

### Exemple 9 :

**Iodure de 1-{5-[4-(3-aminopropylamino)-butylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl}-3H-indolium.** Composé de formule (I) avec R1 = R2 = H, R3 = Me, X = I, n = 3, m = 5,d=0,e=1,b=4,etc=3.

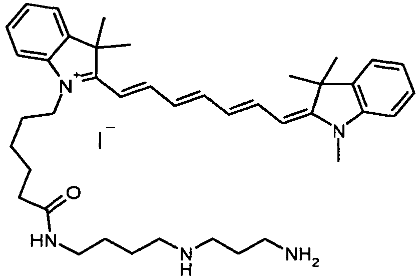

C₄₁H₅₈IN₅O = 763,85.

### Exemple 10 :

**Iodure de 1-{5-[3-(4-aminobutylamino)-propylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl}-3H-indolium.** Composé de formule (I) avec R1 = R2 = H, R3 = Me, X = I, n = 3, m = 5,d=0,e=1,b=3,etc=4.

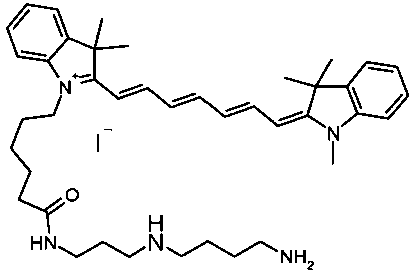

C₄₁H₅₈IN₅O= 763,85.

### Exemple 11 :

**Iodure de 1-[5-(4-aminobutylamino)-pentyl]-3,3-diméthyl-2-{(1E,3E,5E)-7-[1,3,3-triméthyl-1,3-dihydroindol-(2E)-ylidène]-hepta-1,3,5-triényl}-3H-indolium.** Composé de formule (I) avec R1 = R2 = H, R3 = Me, X = I, n = 3, m = 5, d = 1, a = 1, et e=0.

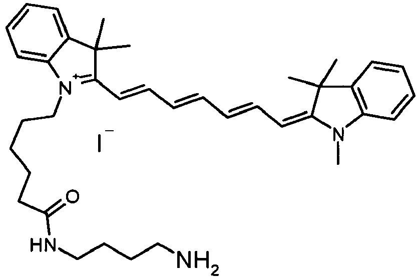

C₃₈H₅₁IN₄O = 706,76.

### II - Etude pharmacologique

Pour la majorité des thérapies anticancéreuses utilisées aujourd'hui en clinique, le biomarqueur permettant d'identifier les tumeurs qui ont une chance de répondre à la thérapie proposée n'existe pas. La présente invention présente des dérivés fluorescents de cyanine polyamine comme sondes de diagnostic pour sélectionner les tumeurs éligibles pour un traitement par un composé anticancéreux vectorisé par le système de transport des polyamines. Contrairement aux sondes revendiquées dans le brevet WO 2009/013360 qui émettent une fluorescence de longueurs d'onde λ ~ 450-520 nm, la présente invention propose des sondes qui fluorescent dans le proche infrarouge (λ ~ 650-900 nm), c'est-à-dire dans la fenêtre spectrale permettant la détection *in vivo* par imagerie de fluorescence. En effet, la fluorescence de longueurs d'onde λ ~ 450-520 nm est difficilement détectable *in vivo* car elle est majoritairement absorbée par les tissus. L'utilisation de telles sondes se fait donc nécessairement *in vitro,* leur application au diagnostic des tumeurs impose donc la réalisation d'une biopsie tumorale. Au contraire, les sondes cyanine de la présente invention (λ ~ 650-900 nm) sont directement applicables *in vivo* par administration au petit animal et pourrait l'être à l'homme, suivie d'une anesthésie gazeuse de l'animal / patient, puis en utilisant l'appareil de détection adapté, l'émission de fluorescence par la sonde qui s'accumule au sein de la tumeur étant enregistrée au cours du temps. Les sondes de la présente invention permettent donc de réaliser un examen direct de la tumeur portée par le sujet, de manière non invasive. Elles pourraient alors permettre également un suivi de la réponse thérapeutique au cours du temps.

Exemple 12 :

### Détection in vivo de la fluorescence émise par la sonde cyanine polyamine 7-7 de l'exemple 7 qui s'accumule au sein de la tumeur portée par l'animal.

### Méthode

### 1 Animaux

6 souris mâles C57B16 saines, âgées de 11 semaines, ont été utilisées pour évaluer la tolérance de l'injection de doses croissantes de sonde de l'exemple 7 (F98942) (25, 50 et 100 µg).

11 souris mâles C57B16 âgées de 10 semaines ont été greffées par voie sous cutanée au niveau de la patte postérieure droite avec 2,5.10⁵ cellules de mélanome murin B16/F10-Luc.

### 2_ La sonde

La sonde F98942 est mise en solution dans du sérum glucosé 5% stérile de manière extemporanée.

### 3_Imagerie de fluorescence

Les souris ont été injectées avec 50µg ou 25µg de F98942 ou du PBS (Phosphate Buffered Saline, tampon phosphate salin) par voie intraveineuse. Elles ont ensuite été anesthésiées par inhalation gazeuse d'isoflurane (3 % isoflurane/oxygène) avant l'examen par imagerie de fluorescence 24 et 48 h post-injection. Après 48 h, les tumeurs ont été prélevées et analysées par imagerie de fluorescence.

### Résultats

L'injection de la sonde à des souris saines est bien tolérée quelle que soit la dose. La fluorescence de la sonde F98942 est détectée au niveau de la tumeur B16F10 *in vivo* comme montré dans le tableau suivant et sur la figure 1.

| **Fixation tumorale** | | | |
|---|---|---|---|
| **Souris** | **F98942 (µg)** | **Fluorescence à t = 24h [p/s/cm²/sr]/[µW/cm²]** | **Moyenne [p/s/cm²/sr]/[µW/cm²]** |
| 1 | 50 | 3,22.10⁷ | 3,14. 10⁷ |
| 2 | 50 | 3,08.10⁷ | |
| 3 | 50 | 3,12_{.}10⁷ | |
| 4 | 50 | 3,01.10⁷ | |
| 5 | 25 | 2,10.10⁷ | 1,81.10⁷ |
| 6 | 25 | 1,52.10⁷ | |
| 7 | 0 | 1,01.10⁶ | 1,02.10⁶ |
| 8 | 0 | 1,02.10⁶ | |

### Exemple 13 (comparatif)

### Détection in vivo de la fluorescence émise par la sonde comparative suivante :

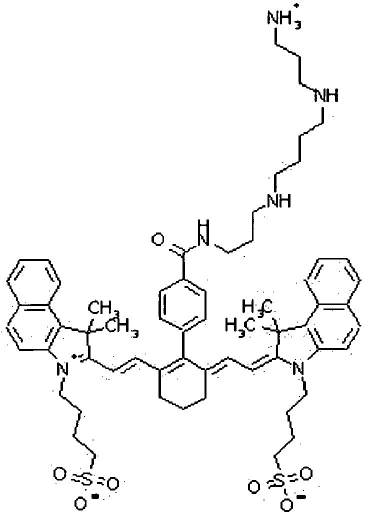

Le même test que celui de l'exemple 12 a été réalisé avec la sonde comparative décrite ci-dessus. Toutefois, ce composé n'a pu être détecté puisqu'il avait un niveau de fluorescence trop faible.

## Revendications

1. Composé de formule (I) suivante : 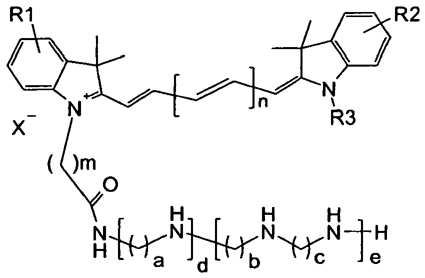 dans laquelle :
- R1 et R2 représentent chacun un atome d'hydrogène,
- R3 représente un groupe C₁₋₆alkyle éventuellement substitué par un groupe -SO₃H,
- n vaut 1 ou 2,
- m vaut 3, 4, 5 ou 6,
- a, b, et c valent, indépendamment les uns des autres, 2, 3, 4, ou 5,
- d et e valent, indépendamment l'un de l'autre, 0 ou 1, à la condition qu'ils ne valent pas tous les deux simultanément 0, et
- X représente un atome d'halogène,
ou un sel pharmaceutiquement acceptable de celui-ci.

2. Composé selon la revendication 1, **caractérisé en ce que** R3 représente un groupe C₁₋₆alkyle.

3. Composé selon l'une quelconque des revendications 1 et 2, **caractérisé en ce que** a, b et c valent, indépendamment les uns des autres, 3 ou 4.

4. Composé selon l'une quelconque des revendications 1 à 3, **caractérisé en ce que** m vaut 5.

5. Composé selon l'une quelconque des revendications 1 à 4, **caractérisé en ce qu'**il est choisi parmi :
1) iodure de 1-(6-(3-(4-(3-aminopropylamino)butylamino)propylamino)-6-oxohexyl) 2-((1*E*,3*E*,5*E*)-5-(1-méthyl-3,3-diméthylindolin-2-ylidène)penta-1,3-diényl)-3,3-diméthyl-3*H*-indolium,
2) iodure de 1-{5-[4-(3-aminopropylamino)-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
3) iodure de 1-{5-[3-(4-aminobutylamino)-propylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
4) iodure de 1-{5-[4-amino-butylcarbamoyl]-pentyl}-3,3-diméthyl-2-{(1*E*,3*E*)-5-[1,3,3-triméthyl-1,3-dihydroindol-(2E)-ylidène]-penta-1,3-diényl}-3H-indolium,
5) bromure de 1-(6-(3-(4-(3-aminopropylamino)butylamino)propylamino)-6-oxohexyl)2-((1*E*,3*E*,5*E*)-5-(1-butyl-3,3-diméthylindolin-2-ylidène)penta-1,3-diényl)-3,3-diméthyl-3*H*-indolium,
6) bromure de 1-{5-[4-(3-aminopropylamino)-butylcarbamoyl]-pentyl}-3,3-diméthyl-2-{(1*E*,3*E*)-5-[1-butyl-3,3-diméthyl-1,3-dihydroindol-(2E)-ylidène]-penta-1,3-diényl}-3H-indolium,
7) bromure de 1-{5-[4-amino-butylcarbamoyl]-pentyl}-3,3-diméthyl-2-{(1*E*,3*E*)-5-[1-butyl-3,3-diméthyl-1,3-dihydroindol-(2E)-ylidène]-penta-1,3-diényl}-3H-indolium,
8) iodure de 1-(5-{3-[4-(3-aminopropylamino)butylamino]-propylcarbamoyl}-pentyl)-2-{(1E,3E,5E)-7-[1,3,3-triméthyl-1,3-dihydroindol-(2E)-ylidène]-hepta-1,3,5-triényl}-3,3-diméthyl-3H-indolium,
9) iodure de 1-{5-[4-(3-aminopropylamino)-butylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl}-3H-indolium,
10) iodure de 1-{5-[3-(4-aminobutylamino)-propylcarbamoyl]-pentyl}-3,3-diméthyl-2-{(1E,3E,5E)-7-[1,3,3-triméthyl-1,3-dihydroindol-(2E)-ylidène]-hepta-1,3,5-triényl}-3H-indolium,
11) iodure del-[5-(4-aminobutylamino)-pentyl]-3,3-diméthyl-2-{(1E,3E,5E)-7-[1,3,3-triméthyl-1,3-dihydroindol-(2E)-ylidène]-hepta-1,3,5-triényl}-3H-indolium,
12) bromure de 1-(5-{3-[4-(3-aminopropylamino)butylamino]-propylcarbamoyl}-pentyl)-2-{(1E,3E,5E)-7-[1-butyl-3,3-diméthyl-1,3-dihydroindol-(2E)-ylidène]-hepta-1,3,5-triényl -3,3-diméthyl-3H-indolium,
13) bromure de 1-{S-[4-(3-aminopropylamino)-butylcarbamoyl]-pentyl}-3,3-diméthyl-2-{(1E,3E,5E)-7-[1-butyl-3,3-diméthyl-1,3-dihydroindol-(2E)-ylidène]-hepta-1,3,5-triényl}-3 H-indolium,
14) bromure de 1-{S-[3-(4-aminobutylamino)-propylcarbamoyl]-pentyl}-3,3-diméthyl-2-{(1E,3E,5E)-7-[1-butyl-3,3-diméthyl-1,3-dihydroindol-(2E)-ylidène]-hepta-1,3,5-triényl}-3H-indolium, et
15) bronure de 1-[5-(4-aminobutylcarbamoyl)-pentyl)-2-{(1E,3E,5E)-7-[1-butyl-3,3-diméthyl-1,3-dihydroindol-(2E)-ylidène]-hepta-1,3,5-triényl}-3,3-diméthyl-3H-indolium,
ou un sel pharmaceutiquement acceptable de ceux-ci, tel qu'un sel d'addition d'acide chlorhydrique.

6. Composition diagnostique comprenant au moins un composé de formule (I) selon l'une quelconque des revendications 1 à 5 ou un sel pharmaceutiquement acceptable de celui-ci et au moins un excipient pharmaceutiquement acceptable.

7. Utilisation d'un composé de formule (I) selon l'une quelconque des revendications 1 à 5 ou d'un sel pharmaceutiquement acceptable de celui-ci comme sonde de diagnostic *in vitro* pour imagerie médicale.

8. Utilisation selon la revendication 7, **caractérisée en ce que** l'imagerie médicale est de l'imagerie par fluorescence.

9. Composé de formule (I) selon l'une quelconque des revendications 1 à 5 ou d'un sel pharmaceutiquement acceptable de celui-ci pour son utilisation dans le diagnostic *in vivo* d'une tumeur cancéreuse exprimant le système de transport des polyamines.

10. Composé pour utilisation selon la revendication 9, **caractérisé en ce que** le diagnostic est réalisé par imagerie médicale par fluorescence.

11. Procédé de synthèse d'un composé de formule (I) selon l'une quelconque des revendications 1 à 5 ou d'un sel pharmaceutiquement acceptable comprenant les étapes successives suivantes :
(i) couplage entre un composé de formule (VII) suivante : 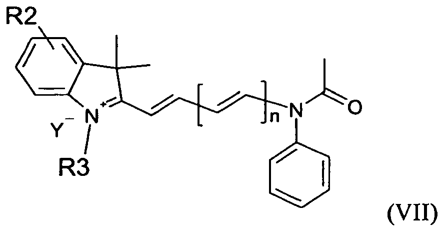 pour laquelle R2, R3 et n sont tels que définis à la revendication 1, et Y représente un atome d'halogène,
avec un composé de formule (V) suivante : 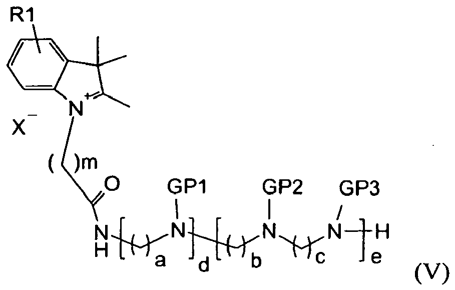 pour laquelle R1, X, m, a, b, c, d et e sont tels que définis à la revendication 1 et GP1, GP2 et GP3, identiques ou différents, représentent un groupe N-protecteur, pour donner un composé de formule (VIII) suivante : 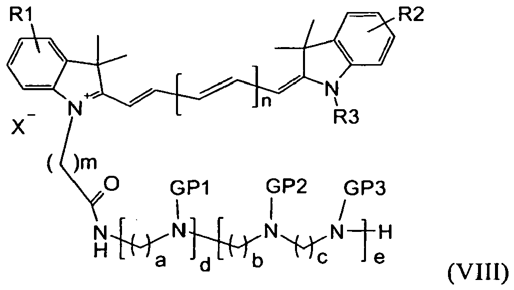 pour laquelle, R1, R2, R3, X, m, n, a, b, c, d, et e sont tels que définis à la revendication 1 et GP1, GP2 et GP3 sont tels que définis précédemment,
(ii) déprotection des fonctions amines protégées par les groupements GP1, GP2 et GP3 dans le composé de formule (VIII) obtenu à l'étape (i) précédente pour donner un composé de formule (I) selon l'invention,
(iii) éventuellement salification du composé de formule (I) obtenu à l'étape (ii) précédente pour obtenir un sel pharmaceutiquement acceptable de celui-ci, et
(iv) séparation du composé de formule (I) ou de son sel pharmaceutiquement acceptable obtenu à l'étape précédente du milieu réactionnel.

12. Procédé selon la revendication 11, **caractérisé en ce que** l'étape (i) est réalisée en présence d'acétate de sodium.

13. Procédé selon la revendication 12, **caractérisé en ce que** l'étape (i) est réalisée dans l'éthanol comme solvant.

14. Procédé selon l'une quelconque des revendications 11 à 13, **caractérisé en ce que** le composé de formule (V) est obtenu par couplage peptidique entre un composé de formule (III) suivante : 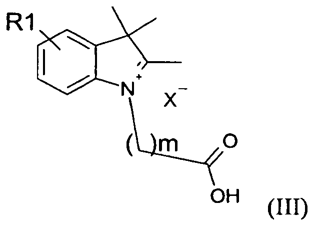 pour laquelle R1, m et X sont tels que définis à la revendication 1, avec une polyamine protégée de formule (IX) suivante : 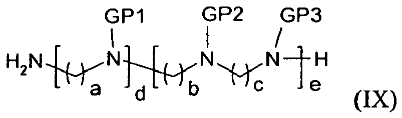 pour laquelle a, b, c, d et e sont tels que définis à la revendication 1 et GP1, GP2 et GP3 sont tels que définis à la revendication 11, ou un sel d'addition d'acide de celle-ci.

15. Procédé selon l'une quelconque des revendications 11 à 14, **caractérisé en ce que** le composé de formule (VII) est obtenu par couplage entre un composé de formule (IV) suivant : 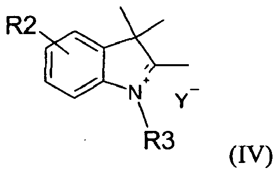 pour laquelle R2 et R3 sont tels que définis à la revendication 1 et Y est tel que défini à la revendication 11,
et un composé de formule (VI) suivante : 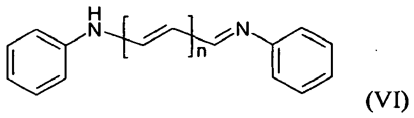 pour laquelle n est tel que défini à la revendication 1, ou un sel d'addition d'acide de celui-ci.

## Patentansprüche

1. Verbindung der folgenden Formel (I): 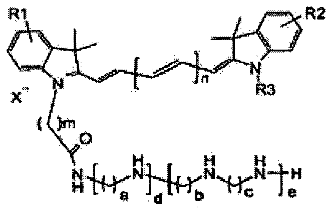 worin:
- R1 und R2 jeweils ein Wasserstoffatom darstellen,
- R3 eine C₁₋₆-Alkylgruppe darstellt, die gegebenenfalls durch eine -SO₃H-Gruppe substituiert ist,
- n gleich 1 oder 2 ist,
- m gleich 3, 4, 5 oder 6 ist,
- a, b, und c, unabhängig voneinander, gleich 2, 3, 4 oder 5 sind,
- d und e, unabhängig voneinander, gleich 0 oder 1 sind, unter der Bedingung, dass nicht beide gleichzeitig gleich 0 sind, und
- X ein Halogenatom darstellt,
oder ein pharmazeutisch unbedenkliches Salz von dieser.

2. Verbindung nach Anspruch 1, **dadurch gekennzeichnet, dass** R3 eine C₁₋₆-Alkylgruppe darstellt.

3. Verbindung nach einem der Ansprüche 1 und 2, **dadurch gekennzeichnet, dass** a, b, und c, unabhängig voneinander, gleich 3 oder 4 sind.

4. Verbindung nach einem der Ansprüche 1 bis 3, **dadurch gekennzeichnet, dass** m gleich 5 ist.

5. Verbindung nach einem der Ansprüche 1 bis 4, **dadurch gekennzeichnet, dass** sie gewählt ist aus:
1) 1-(6-(3-(4-(3-Aminopropylamino)butylamino)propylamino)-6-oxohexyl) 2-((1*E*,3*E*,5*E*)-5-(1-methyl-3,3-dimethylindolin-2-yliden)penta-1,3-dienyl)-3,3-dimethyl-3H-Indoliumiodid,
2) 1-{5-[4-(3-Aminopropylamino)-butylcarbamoyl]-pentyl}-3,3-dimethyl-2{(1*E*,3*E*)-5-[1,3,3-trimethyl-1,3-dihydroindol-(2*E*)-yliden]-penta-1,3-dienyl}-3H-Indoliumiodid,
3) 1-{5-[3-(4-Aminobutylamino)-propylcarbamoyl]-pentyl}-3,3-dimethyl-2{(1*E*,3*E*)-5-[1,3,3-trimethyl-1,3-dihydroindol-(2*E*)-yliden]-penta-1,3-dienyl}-3-Indoliumiodid,
4) 1-{5-[4-Amino-butylcarbamoyl]-pentyl}-3,3-dimethyl-2{(1*E*,3*E*)-5-[1,3,3-trimethyl-1,3-dihydroindol-(2*E*)-yliden]-penta-1,3-dienyl}-3H-Indoliumiodid,
5) 1-(6-(3-(4-(3-Aminopropylamino)butylamino)propylamino)-6-oxohexyl)2-((1*E*,3*E*,5*E*)-5-(1-butyl-3,3-dimethylindolin-2-yliden)-penta-1,3-dienyl)-3,3-dimethyl-3H-Indoliumbromid,
6) 1-{5-[4-(3-Aminopropylamino)-butylcarbamoyl]-pentyl}-3,3-dimethyl-2-{(1*E*,3*E*)-5-[1-butyl-3,3-dimethyl-1,3-dihydroindol-(2*E*)-yliden]-penta-1,3-dienyl)-3H-Indoliumbromid,
7) 1-{5-[4-Amino-butylcarbamoyl]-pentyl}-3,3-dimethyl-2-{(1*E*,3*E*)-5-[1-butyl-3,3-dimethyl-1,3-dihydroindol-(2*E*)-yliden]-penta-1,3-dienyl}-3H-Indoliumbromid,
8) 1-(5-{3-[4-(3-Aminopropylamino)butylamino]-propylcarbamoyl}-pentyl)2-{(1*E*,3*E*,5*E*)-7-[1,3,3-dimethyl-1,3-dihydroindol-(2*E*)-yliden]-hepta-1,3,5-trienyl}-3,3-dimethyl-3H-Indoliumiodid,
9) 1-{5-[4-(3-Aminopropylamino)-butylcarbamoyl]-pentyl}-3,3-dimethyl-2{(1*E*,3*E*,5*E*)-7-[1,3,3-trimethyl-1,3-dihydroindol-(2*E*)-yliden]-hepta-1,3,5-trienyl}-3H-Indoliumiodid,
10) 1-{5-[3-(4-Aminobutylamino)-propylcarbamoyl]-pentyl}-3,3-dimethyl-2{(1*E*,3*E*,5*E*)-7-[1,3,3-dimethyl-1,3-dihydroindol-(2*E*)-yliden]-hepta-1,3,5-trienyl}-3H-Indoliumiodid,
11) 1-[5-(4-Aminobutylamino)-pentyl]-3,3-dimethyl-2-{(1*E*,3*E*,5*E*)-7-[1,3,3-trimethyl-1,3-dihydroindol-(2*E*)-yliden]-hepta-1,3,5-trienyl}-3H-Indoliumiodid,
12) 1-(5-{3-[4-(3-Aminopropylamino)butylamino]-propylcarbamoyl}pentyl)-2-{(1*E*,3*E*,5*E*)-7-[1-butyl-3,3-dimethyl-1,3-dihydroindol-(2*E*)-yliden]-hepta-1,3,5-trienyl}-3,3-dimethyl-3H-Indoliumbromid,
13) 1-{5-[4-(3-Aminopropylamino)-butylcarbamoyl]-pentyl}-3,3-dimethyl-2-{(1*E*,3*E*,5*E*)-7-[1-butyl-3,3-dimethyl-1,3-dihydroindol-(2*E*)-yliden]-hepta-1,3,5-trienyl)-3H-Indoliumbromid,
14) 1-{5-[3-(4-Aminobutylamino)-propylcarbamoyl]-pentyl}-3,3-dimethyl-2-{(1*E*,3*E*,5*E*)-7-[1-butyl-3,3-dimethyl-1,3-dihydroindol-(2E)-yliden]-hepta-1,3,5-trienyl}-3H-Indoliumbromid und
15) 1-[5-(4-Aminobutylcarbamoyl)-pentyl]-2-{(1*E*,3*E*,5*E*)-7-[1-butyl-3,3-dimethyl-1,3-dihydroindol-(2*E*)-yliden]-hepta-1,3,5-trienyl}-3,3-dimethyl-3 Indoliumbromid,
oder ein pharmazeutisch unbedenkliche Salz von diesen, wie z. B. ein Chlorwasserstoffsäureadditionssalz.

6. Diagnosezusammensetzung umfassend mindestens eine Verbindung der Formel (I) nach einem der Ansprüche 1 bis 5 oder ein pharmazeutisch unbedenkliches Salz von dieser und mindestens einen pharmazeutisch unbedenklichen Träger.

7. Verwendung einer Verbindung der Formel (I) nach einem der Ansprüche 1 bis 5 oder eines pharmazeutisch unbedenklichen Salzes von dieser als *Invitro*-Diagnosesonde für medizinische Bildgebung.

8. Verwendung nach Anspruch 7, **dadurch gekennzeichnet, dass** die medizinische Bildgebung Fluoreszenzbildgebung ist.

9. Verbindung der Formel (I) nach einem der Ansprüche 1 bis 5 oder eines pharmazeutisch unbedenklichen Salzes von dieser für deren bzw. dessen Verwendung in der *In-vivo*-Diagnose eines Krebstumors, der das Polyamintransportsystem exprimiert.

10. Verbindung zur Verwendung nach Anspruch 9, **dadurch gekennzeichnet, dass** der Diagnose durch medizinische Fluoreszenzbildgebung erfolgt.

11. Verfahren zur Synthese einer Verbindung der Formel (I) nach einem der Ansprüche 1 bis 5 oder eines pharmazeutisch unbedenklichen Salzes, umfassend die folgenden aufeinanderfolgenden Schritte:
(i) Kupplung zwischen einer Verbindung der folgenden Formel (VII): 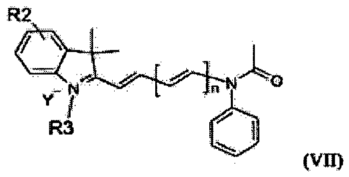 worin R2, R3 und n wie in Anspruch 1 definiert sind und Y ein Halogenatom darstellt,
und einer Verbindung der folgenden Formel (V): 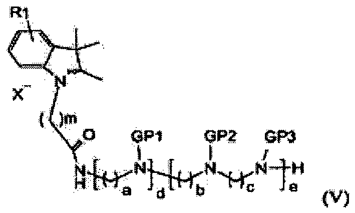 worin R1, X, m, a, b, c, d und e wie in Anspruch 1 definiert sind und GP1, GP2 und GP3, die identisch oder unterschiedlich sind, eine N-Schutzgruppe darstellen, um eine Verbindung der folgenden Formel (VIII) zu ergeben: 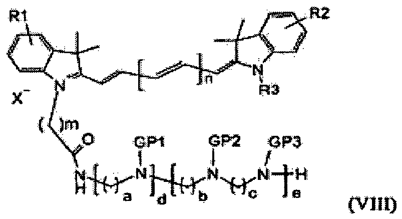 worin R1, R2, R3, X, m, n, a, b, c, d und e wie in Anspruch 1 definiert sind und GP1, GP2 und GP3 wie oben definiert sind,
(ii) Entschützung der durch die Gruppen GP1, GP2 und GP3 geschütz-ten Aminofunktionen in der im obigen Schritt (i) erhaltenen Verbindung der Formel (VIII), um eine Verbindung der Formel (I) nach der Erfindung zu ergeben,
(iii) gegebenenfalls Salzbildung der im vorhergehenden Schritt (ii) erhaltenen Verbindung der Formel (I), um ein pharmazeutisch unbedenkliches Salz von dieser zu erhalten, und
(iv) Trennen der im vorhergehenden Schritt erhaltenen Verbindung der Formel (I) oder ihres im obigen Schritt erhaltenen Salzes von dem Reaktionsmedium.

12. Verfahren nach Anspruch 11, **dadurch gekennzeichnet, dass** Schritt (i) in Gegenwart von Natriumacetat erfolgt.

13. Verfahren nach Anspruch 12, **dadurch gekennzeichnet, dass** Schritt (i) in Ethanol als Lösemittel erfolgt.

14. Verfahren nach einem der Ansprüche 11 bis 13, **dadurch gekennzeichnet, dass** die Verbindung der Formel (V) erhalten wird durch Peptidkupplung zwischen einer Verbindung der folgenden Formel (III): 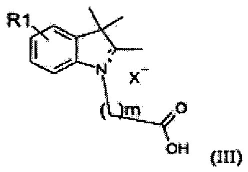 worin R1, m und X wie in Anspruch 1 definiert sind,
und einem geschütz-ten Polyamin der folgenden Formel (IX): 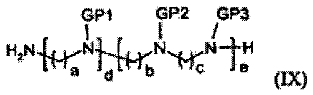 worin a, b, c, d und e wie in Anspruch 1 definiert sind und GP1, GP2 und GP3 wie in Anspruch 11 definiert sind, oder einem Säureaddi-tionssalz von dieser.

15. Verfahren nach einem der Ansprüche 11 bis 14, **dadurch gekennzeichnet, dass** die Verbindung der Formel (VII) erhalten wird durch Kupplung zwischen einer Verbindung der folgenden Formel (IV): 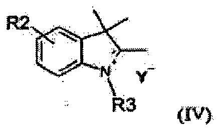 worin R2 und R3 wie in Anspruch 1 definiert sind und Y wie in Anspruch 11 definiert ist,
und einer Verbindung der folgenden Formel (VI): 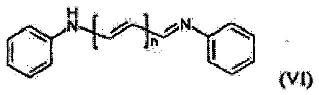 worin n wie in Anspruch 1 definiert ist, oder einem Säureadditionssalz von dieser.

## Claims

1. A compound having the following formula (I): 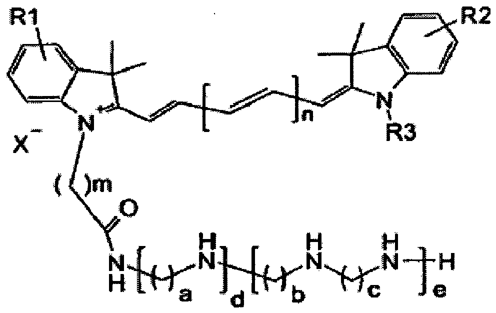 wherein:
- R1 and R2 each represent a hydrogen atom,
- R3 represents a C₁₋₆alkyl group optionally substituted with a -SO₃H group,
- n equals 1 or 2,
- m equals 3, 4, 5 or 6,
- a, b, and c equal, independently of each other, 2, 3, 4, or 5,
- d and e equal, independently of one another, 0 or 1, on the condition that they are not both simultaneously equal to 0, and
- X represents a halogen atom,
or a pharmaceutically acceptable salt thereof.

2. The compound according to claim 1, **characterised in that** R3 represents a C₁₋₆alkyl group.

3. The compound according to any of claims 1 and 2, **characterised in that** a, b and c equal, independently of each other, 3 or 4.

4. The compound according to any of claims 1 to 3, **characterised in that** m equals 5.

5. The compound according to any of claims 1 to 4, **characterised in that** it chosen from:
1) 1-(6-(3-(4-(3-aminopropylamino)butylamino)propyl-amino)-6-oxohexyl)-2-((1*E*,3*E*,5*E*)-5-(1-methyl-3,3-dimethylindolin-2-ylidene)penta-1,3-dienyl)-3,3-dimethyl-3*H*-indolium iodide,
2) 1-{5-[4-(3-aminopropylamino)-butylcarbamoyl]-pentyl}-3,3-dimethyl-2-{(1*E*,3*E*)-5-[1,3,3-trimethyl-1,3-dihydroindol-(2E)-ylidene]-penta-1,3-dienyl}-3H-indolium iodide,
3) 1-{5-[3-(4-aminobutylamino)-propylcarbamoyl]-pentyl}-3,3-dimethyl-2-{(1*E*,3*E*)-5-[1,3,3-trimethyl-1,3-dihydroindol-(2E)-ylidene]-penta-1,3-dienyl}-3H-indolium iodide,
4) 1-{5-[4-amino-butylcarbamoyl]-pentyl}-3,3-dimethyl-2-{(1*E*,3*E*)-5-[1,3,3-trimethyl-1,3-dihydroindol-(2E-ylidene]-penta-1,3-dienyl}-3H-indolium iodide,
5) 1-(6-(3-(4-(3-aminopropylamino)butylamino)propyl-amino)-6-oxohexyl)2-((1*E*, 3*E*, 5*E*)-5-(1-butyl-3,3-dimethylindolin-2-ylidene)penta-1,3-dienyl)-3,3-dimethyl-3*H*-indolium bromide,
6) 1-{5-[4-(3-aminopropylamino)-butylcarbamoyl]-pentyl}-3,3-dimethyl-2-{(1*E*, 3*E*)-5-[1-butyl-3,3-dimethyl-1,3-dihydroindol-(2E)-ylidene]-penta-1,3-dienyl}-3H-indolium bromide,
7) 1-{5-[4-amino-butylcarbamoyl]-pentyl}-3,3-dimethyl-2-{(1*E*, 3*E*)-5-[1-butyl-3,3-dimethyl-1,3-dihydroindol-(2E)-ylidene]-penta-1,3-dienyl}-3H-indolium bromide,
8) 1-(5-{3-[4-(3-aminopropylamino)butylamino]-propyl-carbamoyl}-pentyl)-2-{(1E,3E,5E)-7-[1,3,3-trimethyl-1,3-dihydroindol-(2E)-ylidene]-hepta-1,3,5-trienyl}-3,3-dimethyl-3H-indolium iodide,
9) 1-{5-[4-(3-aminopropylamino)-butylcarbamoyl]-pentyl}-3,3-dimethyl-2-{(1E, 3E, 5E)-7-[1,3,3-trimethyl-1,3-dihydroindol-(2E)-ylidene]-hepta-1,3,5-trienyl}-3H-indolium iodide,
10) 1-{5-[3-(4-aminobutylamino)-propylcarbamoyl]-pentyl}-3,3-dimethyl-2-{(1E,3E,5E)-7-[1,3,3-trimethyl-1,3-dihydroindol-(2E)-ylidene]-hepta-1,3,5-trienyl}-3H-indolium iodide,
11) 1-[5-(4-aminobutylamino)-pentyl]-3,3-dimethyl-2-{(1E, 3E, 5E)-7-[1,3,3-trimethyl-1,3-dihydroindol-(2E)-ylidene]-hepta-1,3,5-trienyl}-3H-indolium iodide,
12) 1-(5-{3-[4-(3-aminopropylamino)butylamino]-propylcarbamoyl}-pentyl)-2-{(1E, 3E, 5E)-7-[1-butyl-3,3-dimethyl-1,3-dihydroindol-(2E)-ylidene]-hepta-1,3,5-trienyl}-3,3-dimethyl-3H-indolium bromide,
13) 1-{5-[4-(3-aminopropylamino)-butylcarbamoyl]-pentyl}-3,3-dimethyl-2-{(1E, 3E, 5E)-7-[1-butyl-3,3-dimethyl-1,3-dihydroindol-(2E)-ylidene]-hepta-1,3,5-trienyl}-3H-indolium bromide,
14) 1-{5-[3-(4-aminobutylamino)-propylcarbamoyl]-pentyl}-3,3-dimethyl-2-{(1E, 3E, 5E)-7-[1-butyl-3,3-dimethyl-1,3-dihydroindol-(2E)-ylidene]-hepta-1,3,5-trienyl}-3H-indolium bromide, and
15) 1-[5-(4-aminobutylcarbamoyl)-pentyl]-2-{(1E,3E, 5E)-7-[1-butyl-3,3-dimethyl-1,3-dihydroindol-(2E)-ylidene]-hepta-1,3,5-trienyl}-3,3-dimethyl-3H-indolium bromide,
or a pharmaceutically acceptable salt thereof, such as a hydrochloric acid addition salt.

6. A diagnostic composition comprising at least one compound having formula (I) according to any of claims 1 to 5 or a pharmaceutically acceptable salt thereof and at least one pharmaceutically acceptable excipient.

7. Use of a compound having formula (I) according to any of claims 1 to 5 or a pharmaceutically acceptable salt thereof as an *in vitro* diagnostic probe for medical imaging.

8. Use according to claim 7, **characterised in that** the medical imaging is fluorescence imaging.

9. A compound having formula (I) according to any of claims 1 to 5 or a pharmaceutically acceptable salt thereof for use for *in vivo* diagnostics of a tumour expressing the polyamine transport system.

10. A compound for use according to claim 9, **characterised in that** the diagnostics are performed by fluorescence medical imaging.

11. A process for synthesising a compound having formula (I) according to any of claims 1 to 5 or a pharmaceutically acceptable salt thereof comprising the following successive steps:
(i) coupling between a compound having the following formula (VII): 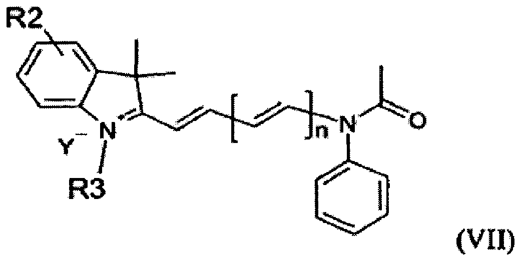 wherein R2, R3 and n are as defined in claim 1, and Y represents a halogen atom,
with a compound having the following formula (V): 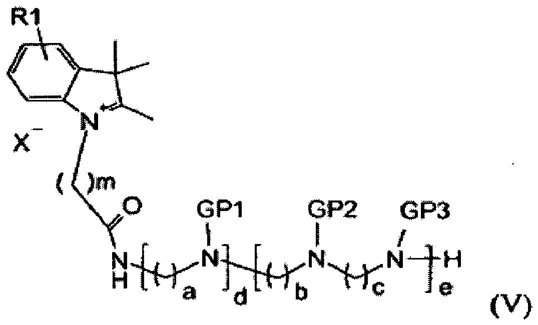 wherein R1, X, m, a, b, c, d and e are as defined in claim 1 and GP1, GP2 and GP3, identical or different, represent an N-protecting group,
to obtain a compound having the following formula (VIII): 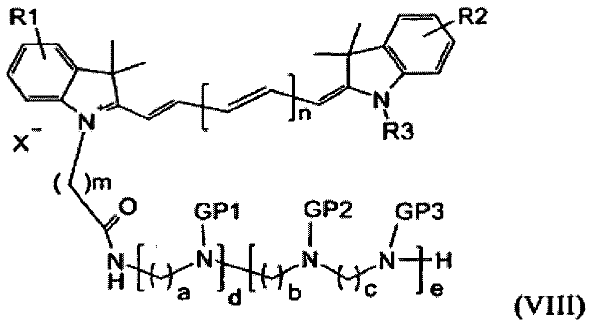 wherein, R1, R2, R3, X, m, n, a, b, c, d, and e are as defined in claim 1 and GP1, GP2 and GP3 are as defined above,
(ii) deprotecting the amine functions protected by the GP1, GP2 and GP3 groups in the compound having formula (VIII) obtained in step (i) above to obtain a compound having formula (I) according to the invention,
(iii) optionally salifying the compound having formula (I) obtained in step (ii) above to obtain a pharmaceutically acceptable salt thereof, and
(iv) separating the compound having formula (I) or the pharmaceutically acceptable salt thereof obtained in the previous step from the reaction medium.

12. The process according to claim 11, **characterised in that** step (i) is carried out in the presence of sodium acetate.

13. The process according to claim 12, **characterised in that** step (i) is carried out in ethanol as solvent.

14. The process according to any of claims 11 to 13, **characterised in that** the compound having formula (V) is obtained by peptide coupling between a compound having the following formula (III): 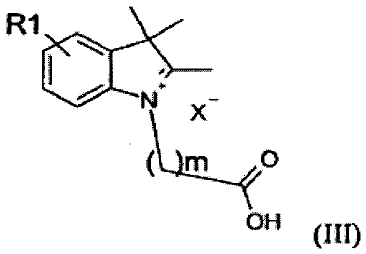 wherein R1, m and X are as defined in claim 1,
with a protected polyamine having the following formula (IX) : 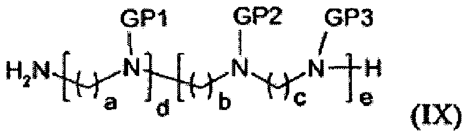 wherein a, b, c, d and e are as defined in claim 1 and GP1, GP2 and GP3 are as defined in claim 11, or an acid addition salt thereof.

15. The process according to any of claims 11 to 14, **characterised in that** the compound having formula (VII) is obtained by coupling between a compound having the following formula (IV): 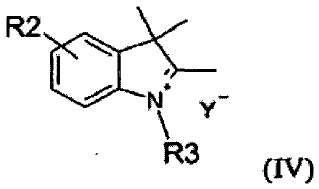 wherein R2 and R3 are as defined in claim 1 and Y is as defined in claim 11,
and a compound having the following formula (VI): 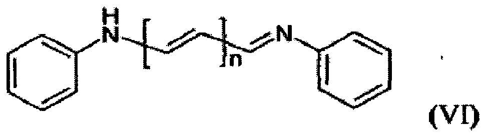 wherein n is as defined in claim 1, or an acid addition salt thereof.
